# EUROPEAN PATENT APPLICATION

(11) **EP 3 871 623 A1**
(43) Date of publication of application: **01.09.2021**
(21) Application number: 20214220.4
(22) Date of filing: 10.10.2008
(51) Int. Cl.: A61B 17/425, A61N 1/36

(54) **DEVICE AND METHOD FOR EMBRYO CONTROL**

(30) Priority: 11.10.2007 US 96071507 P; 11.10.2007 US 96071607 P; 19.10.2007 US 96091807 P
(62) Divisional of application: 08837652.0
(71) Applicant: Implantica Patent Ltd, 223 70 Lund (SE)
(72) Inventor: Forsell, Peter, 223 70 Lund (SE)
(74) Representative: AWA Sweden AB

(57) **Abstract**

A system for treating a female patient to promote pregnancy comprising a restriction device adapted to postoperatively restrict and release an oviduct of the patient.

## Description

### FIELD OF THE INVENTION

The present invention relates generally to medical devices and treatment and, more particularly, to a device and a method for embryo control of a female patient.

### BACKGROUND OF THE INVENTION

Many women have difficulties getting pregnant. Older age when trying to get pregnant with lower fertility rate is one main reason.

Artificial insemination is one way to promote pregnancy and this way has increased dramatically the last few years. However, this method is not always easy and safe and is furthermore very expensive, particularly when repeated, which often is the case.

Although uterus is prepared to receive an embryo for approximately three days it seems that the right timing is of outmost importance when fertility goes down.

### BRIEF SUMMARY OF THE INVENTION

The object of the present invention is to provide a device and a method for embryo control of a female patient.

The inventive device allows the egg from the ovary to be retained in the oviduct for a couple of days to be able to achieve the right timing for egg release and thereby increasing the likelihood to get pregnant with up to 10 times. A respective restriction pregnancy device is placed on the two oviducts to restrict and release the oviduct, thereby effecting the above mentioned retaining of the egg.

In accordance with a first aspect of the present invention, there is provided a device for treating a female patient to promote pregnancy comprising a device adapted to postoperatively restrict and release an oviduct of the patient. To reduce any possible risk of the egg being to the wall of the oviduct the restriction device may be combined with a movement device. Such a device may comprise a vibrating device or a stimulating device. The stimulation device may create a peristaltic like wave in the oviduct. For example, a vibrating device may be provided, which causes vibrations in a hydraulic fluid or mechanically causes vibrations in the wall of the oviduct. Another embodiment is an electrical stimulation device causing contractions or stimulation waves in the upstream direction of the oviduct. The device may also be replaced by a device not permitting the flow of the egg down to the uterus but without any full restriction. This may be created by using a device causing a peristaltic like wave in the oviduct in the direction of the ovary, thus without fully closing the oviduct instead preventing further transport of the egg down to the uterus by the wave, but still allowing the sperm to reach the egg. Any possible device without restriction at all or in any combinations of partly restriction or vibration or stimulation may be used. This will then allow the egg to be accumulated in the oviduct and allowing conception to take place over a longer period of time.

In accordance with a second aspect of the invention, there is provided a method of promoting pregnancy of a female patient, comprising the steps of restricting an oviduct of the patient to provide a restriction to accumulate at least one egg released from the ovary in the oviduct for a predetermined period of time, and releasing the restriction to admit the at least on egg in the oviduct to allow a transport of the at least one egg to the uterus.

The restriction device is adapted to provide a restriction of the oviduct to accumulate at least one egg released from the ovary in the oviduct and to provide a release of the oviduct after accumulating the egg normally up to three days when pregnancy is wanted to achieve right timing for pregnancy.

The restriction device may be adapted to be adjusted from outside the patients body to restrict and release the oviduct passageway, preferable adjusted from outside the patients body noninvasively.

It may also be adjusted by manual manipulation or adapted to be adjusted by electrical or magnetic power or adapted to be adjusted by hydraulic power. The hydraulic power may comprising at least one subcutaneously placed reservoir controlled by the patient.

The restriction device may of course preferable be adapted to be adjusted reversible.

The system is adapted to provide a restriction to accumulate at least one egg released from the ovary in the oviduct for a predetermined period of time, and releasing the restriction when convenient for the patient to increase the likelihood to get pregnant. The predetermined period of time is adapted to be between 0 and 2 days repeated very month until pregnancy is achieved or preferable a predetermined period between 8 and 12 hours.

All embodiments and features described below may if possible be used for both, be adapted to be used with the apparatus, and being used with any of the methods described below.

### Flow restriction

The device of the present invention is well suited for the controlling the flow of eggs into the uterus of a female patient. Basically the device could be performed in eight different principle ways:
- Hydraulic restrictions,
- Mechanical restrictions,
- Combination of any hydraulic or mechanical restriction device with a stimulation device for restricting, and
- Stimulation device alone,
- Vary the restriction area. Any of these in any combination could be used to over time vary the restriction area of the oviduct from one portion to another one and later back again. Several different areas could be involved in such a system to vary the restriction portion/area. This would allow the oviduct to recover the restricted area still keeping the restriction over a longer period.
- Using a moving device. When the restriction area being moved between different areas, specially when the restriction is moved upstream towards the ovary, it may be convenient to use a moving device, to avoid any egg being squeezed in a new upstream restriction area and by mistake being released for further downstream transportation when the restriction being released and moved further upstream. The moving device may be adapted to create a movement of the oviduct wall to create a movement of any egg placed in the new upcoming restricted area. This could be caused by any of the above mentioned restriction devices, mechanical, hydraulic or stimulation device alone or in any combination, but may also be a separate device which also may be a mechanical, a hydraulic or a stimulation device. In one embodiment the moving device cause vibrations.
- Peristaltic like wave movement of the oviduct wall towards the ovary. Another principle is to stop the egg reaching the uterus by creating peristaltic wave like movements of the oviduct wall.

Such peristaltic wave like movements upstream direction towards the ovary may stop flow of any egg towards the uterus without fully restricting the oviduct to allow any sperm to pass and also
- Peristaltic like wave movement of the oviduct wall towards the uterus by being able to cause movement of the egg down to uterus with a down stream peristaltic wave.

These areas will be outlined below.

### Hydraulic restriction

Where the operation device hydraulically operates the constriction device of the restriction or combined restriction/stimulation unit (d), it includes hydraulic means for adjusting the device.

In an embodiment of the invention, the hydraulic means comprises a reservoir and an expandable/contractible cavity in the constriction device, wherein the operation device distributes hydraulic fluid from the reservoir to expand the cavity, and distributes hydraulic fluid from the cavity to the reservoir to contract the cavity. The cavity may be defined by a balloon of the constriction device that abuts the tissue wall portion of the patient's organ, so that the patient's wall portion is constricted upon expansion of the cavity and released upon contraction of the cavity.

Alternatively, the cavity may be defined by a bellows that displaces a relatively large contraction element of the constriction device, for example a large balloon that abuts the wall portion, so that the patient's wall portion is constricted upon contraction of the bellows and released upon expansion of the bellows. Thus, a relatively small addition of hydraulic fluid to the bellows causes a relatively large increase in the constriction of the wall portion. Such a bellows may also be replaced by a suitably designed piston/cylinder mechanism.

Where the hydraulic means comprises a cavity in the constriction device, the apparatus of the invention can be designed in accordance with the options listed below.
1) The reservoir comprises first and second wall portions, and the operation device displaces the first and second wall portions relative to each other to change the volume of the reservoir, such that fluid is distributed from the reservoir to the cavity, or from the cavity to the reservoir.
   1a) The first and second wall portions of the reservoir are displaceable relative to each other by at least one of a magnetic device, a hydraulic device or an electric control device.
2) The apparatus comprises a fluid conduit between the reservoir and the cavity, wherein the reservoir forms part of the conduit. The conduit and reservoir and apparatus are devoid of any non-return valve. The reservoir forms a fluid chamber with a variable volume, and distributes fluid from the chamber to the cavity by a reduction in the volume of the chamber and withdraws fluid from the cavity by an expansion of the volume of the chamber. The apparatus further comprises a motor for driving the reservoir, comprising a movable wall of the reservoir for changing the volume of the chamber.

In a special embodiment of the invention, the operation device comprises a reverse servo operatively connected to the hydraulic means. The term "reverse servo" is to be understood as a mechanism that transfers a strong force acting on a moving element having a short stroke into a weak force acting on another moving element having a long stroke; *i.e.,* the reverse function of a normal servo mechanism. Thus, minor changes in the amount of fluid in a smaller reservoir could be transferred by the reverse servo into major changes in the amount of fluid in a larger reservoir. The reverse servo is particularly suited for manual operation thereof.

Preferably a manually operated reservoir could be placed subcutaneously for manual manipulation thereof.

### Mechanical restriction

Where the operation device mechanically operates the device or the restriction/stimulation unit (d), it may be non-inflatable. Furthermore, the operation device may comprise a servo device, which may include a gearbox. The term "servo device" encompasses the normal definition of a servo mechanism, *i.e.,* an automatic device that controls large amounts of power by means of very small amounts of power, but may alternatively or additionally encompass the definition of a mechanism that transfers a weak force acting on a moving element having a long stroke into a strong force acting on another moving element having a short stroke. Preferably, the operation device operates the constriction device in a non-magnetic and/or non-manual manner. A motor may be operatively connected to the operation device. The operation device may be operable to perform at least one reversible function and the motor may be capable of reversing the function.

### Combination of mechanical or hydraulic device and a stimulation device

The present invention provides an advantageous combination of restriction and stimulation devices, which results in a two-stage influence on the flow of eggs in the lumen of the oviduct. Thus, the constriction device may gently constrict the tissue wall of the oviduct wall by applying a relatively weak force against the wall portion, and the stimulation device may stimulate the constricted wall portion to achieve the desired final influence on the flow in the lumen. The phrase "gently constricting a portion of the tissue wall" is to be understood as restricting the wall portion without substantially hampering the blood circulation in the tissue wall.

In accordance with a first flow restriction option, the control device controls the constriction device to constrict the wall portion, such that flow in the lumen is restricted or stopped, and controls the stimulation device to stimulate the constricted wall portion to cause contraction thereof, such that flow in the lumen is further restricted or more safely stopped. More precisely, a control device may control the stimulation device in a first mode to stimulate the constricted wall portion to further restrict or stop the flow in the lumen and to:
a) control the stimulation device in a second mode to cease the stimulation of the wall portion to increase the flow in the lumen; or
b) control the stimulation and constriction devices in the second mode to cease the stimulation of the wall portion and release the wall portion to restore the flow in the lumen.

Thus both a method for controlling the flow in the lumen and an apparatus adapted to control the flow in the lumen may be implemented according to different embodiments and features in any combination described in this document.

d) Stimulation device alone, which could both 1) restrict by stimulation and 2) also creating peristaltic wave like movements to if a) upstream stop flow without fully restricting the oviduct and also b) being able to cause movement of the egg down to uterus with a down stream peristaltic wave:
Preferably, the stimulation device is adapted to stimulate different areas of the wall portion as the device restricts the wall portion, and the control device controls the stimulation device to intermittently and individually stimulate the areas of the wall portion. This intermittent and individual stimulation of different areas of the wall portion of the oviduct allows tissue of the wall portion to maintain substantially normal blood circulation during the operation of the apparatus of the invention.

The stimulation of different areas may be used both for closing the lumen in a safe way but also be used for creating a peristaltic wave in the oviduct.

### Movement of the egg in the oviduct lumen

In one embodiment the device is adapted to constrict the wall portion to restrict or vary the flow in the lumen, and the control device controls the stimulation device to progressively stimulate the constricted wall portion, in the downstream or upstream direction of the lumen, to cause progressive contraction of the wall portion to move the egg downstreams in the lumen or prevent further transport down of the egg to the uterus.

### Design of control device

The device is preferable controlled from outside the body in a manual way. The device may also be powered. A control device may be supplied. The control device suitably controls the device or the stimulation device or restriction/stimulation unit from outside the patient's body. Preferably, the control device is operable by the patient. For example, the control device may comprise a manually operable switch for switching on and off the constriction/stimulation unit, wherein the switch is adapted for subcutaneous implantation in the patient to be manually or magnetically operated from outside the patient's body. Alternatively, the control device may comprise a hand-held wireless remote control, which is conveniently operable by the patient to switch on and off the constriction/stimulation unit. The wireless remote control may also be designed for application on the patient's body like a wristwatch. Such a wristwatch type of remote control may emit a control signal that follows the patient's body to implanted signal responsive means of the apparatus.

In a preferred embodiment of the invention, the constriction device is adjustable to enable adjustment of the constriction of the wall portion as desired, wherein the control device controls the constriction device to adjust the constriction of the wall portion. The control device may control the constriction and stimulation devices independently of each other, and simultaneously. Optionally, the control device may control the stimulation device to stimulate, or to not stimulate the wall portion while the control device controls the constriction device to change the constriction of the wall portion.

Initially, the constriction device may be calibrated by using the control device to control the stimulation device to stimulate the wall portion, while controlling the constriction device to adjust the constriction of the wall portion until the desired restriction of the flow in the lumen is obtained.

### Regarding Stimulation alone or in combination with a device

The control device may control the stimulation device to stimulate one or more of the areas of the wall portion at a time, for example by sequentially stimulating the different areas. Furthermore, the control device may control the stimulation device to cyclically propagate the stimulation of the areas along the wall portion, preferably in accordance with a determined stimulation pattern. To achieve the desired reaction of the tissue wall during the stimulation thereof, the control device may control the stimulation device to, preferably cyclically, vary the intensity of the stimulation of the wall portion.

In a preferred embodiment of the invention, the control device controls the stimulation device to intermittently stimulate the areas of the wall portion with pulses that preferably form pulse trains. At least a first area and a second area of the areas of the wall portion may be repeatedly stimulated with a first pulse train and a second pulse train, respectively, such that the first and second pulse trains over time are shifted relative to each other. For example, the first area may be stimulated with the first pulse train, while the second area is not stimulated with said second pulse train, and vice versa. Alternatively, the first and second pulse trains may be shifted relative to each other, such that the first and second pulse trains at least partially overlap each other.

The pulse trains can be configured in many different ways. Thus, the control device may control the stimulation device to vary the amplitudes of the pulses of the pulse trains, the duty cycle of the individual pulses of each pulse train, the width of each pulse of the pulse trains, the length of each pulse train, the repetition frequency of the pulses of the pulse trains, the repetition frequency of the pulse trains, the number of pulses of each pulse train, and/or the off time periods between the pulse trains. Several pulse trains of different configurations may be employed to achieve the desired effect.

In case the control device controls the stimulation device to vary the off time periods between pulse trains that stimulate the respective area of the wall portion, it is also possible to control each off time period between pulse trains to last long enough to restore substantially normal blood circulation in the area when the latter is not stimulated during the off time periods.

An electric stimulation device suitably comprises at least one, preferably a plurality of electrical elements, such as electrodes, for engaging and stimulating the wall portion with electric pulses. Optionally, the electrical elements may be placed in a fixed orientation relative to one another. The control device controls the electric stimulation device to electrically energize the electrical elements, one at a time, or groups of electrical elements at a time. Preferably, the control device controls the electric stimulation device to cyclically energize each element with electric pulses. Optionally, the control device may control the stimulation device to energize the electrical elements, such that the electrical elements are energized one at a time in sequence, or such that a number or groups of the electrical elements are energized at the same time. Also, groups of electrical elements may be sequentially energized, either randomly or in accordance with a predetermined pattern.

The electrical elements may form any pattern of electrical elements. Preferably, the electrical elements form an elongate pattern of electrical elements, wherein the electrical elements are applicable on the patient's wall of the organ, such that the elongate pattern of electrical elements extends lengthwise along the wall of the organ, and the elements abut the respective areas of the wall portion. The elongate pattern of electrical elements may include one or more rows of electrical elements extending lengthwise along the wall of the organ. Each row of electrical elements may form a straight, helical or zig-zag path of electrical elements, or any form of path. The control device may control the stimulation device to successively energize the electrical elements longitudinally along the elongate pattern of electrical elements in a direction opposite to, or in the same direction as that of, the flow in the patient's lumen.

In accordance with a preferred embodiment of the invention, the electrical elements form a plurality of groups of elements, wherein the groups form a series of groups extending along the patient's organ in the flow direction in the patient's lumen. The electrical elements of each group of electrical elements may form a path of elements extending at least in part around the patient's organ. In a first alternative, the electrical elements of each group of electrical elements may form more than two paths of elements extending on different sides of the patient's organ, preferably substantially transverse to the flow direction in the patient's lumen. The control device may control the stimulation device to energize the groups of electrical elements in the series of groups in random, or in accordance with a predetermined pattern. Alternatively, the control device may control the stimulation device to successively energize the groups of electrical elements in the series of groups in a direction opposite to, or in the same direction as that of, the flow in the patient's lumen, or in both said directions starting from a position substantially at the center of the constricted wall portion. For example, groups of energized electrical elements may form advancing waves of energized electrical elements, as described above; that is, the control device may control the stimulation device to energize the groups of electrical elements, such that energized electrical elements form two waves of energized electrical elements that simultaneously advance from the center of the constricted wall portion in two opposite directions towards both ends of the elongate pattern of electrical elements.

### Sensor

The control device may control the stimulation device to change the stimulation of the wall portion in response to a sensed physical parameter of the patient or functional parameter of the device. For example, the control device may control the stimulation device to increase the intensity of the stimulation of the wall portion in response to a sensed pressure increase in the oviduct, such that the flow in the oviduct remains stopped. Any sensor for sensing a physical parameter of the patient, such as a pressure in the patient's body that relates to the pressure in the oviduct may be provided, wherein the control device controls the stimulation device in response to signals from the sensor. Such a sensor may for example sense the pressure in the patient's abdomen, the pressure against the implanted constriction device or the pressure on the tissue wall of the bodily organ.

For example, a hormone level sensor may be applied where the present invention is used for controlling flow of the egg.

### Sensor Controlled Restriction and/or Stimulation Device

As mentioned above, the device may comprise at least one implantable sensor, wherein the control device controls the constriction device and/or the stimulation device in response to signals from the sensor. Generally, the sensor directly or indirectly senses at least one physical parameter of the patient, or at least one functional parameter of the device, or at least one functional parameter of a medical implant in the patient.

The system is further preferable adapted to send feedback information from inside the body to the outside thereof to give feed back related to any functional parameter of the device or physical parameter of the patient.

The functional parameter of the device may be correlated to the transfer of energy for charging the internal energy source mentioned in other places.

The functional parameter of the device may be the energy balance, the balance between the energy received and the energy used including accumulated by the device and the energy balance could also include the balance between an energy reception rate and an energy using including accumulating rate.

Many different kinds of sensor for sensing physical parameters may be used.

The control device may comprise an implantable internal control unit that directly controls the constriction device and/or stimulation device in response to signals from the sensor. The control device may further comprise a wireless remote control adapted to set control parameters of the internal control unit from outside the patient without mechanically penetrating the patient. At least one of the control parameters, which is settable by the wireless remote control, is the physical or functional parameter. Suitably, the internal control unit includes the above mentioned clock mechanism, wherein the wireless remote control also is adapted to set the clock mechanism.

Alternatively, the control device may comprise an external control unit outside the patient's body for controlling the constriction device and/or stimulation device in response to signals from the sensor.

### Adjustable Constriction Device

In several alternative embodiments of the invention, the constriction device is adjustable. In these embodiments, there is an operation device for operating the adjustable constriction device to change the constriction of the patient's tissue wall portion, and the constriction and stimulation devices form a constriction/stimulation unit. Preferably, the constriction and stimulation devices of the constriction/stimulation unit are integrated in a single piece suitable for implantation. The constriction device of the unit comprises contact surfaces dimensioned to contact a length of a tissue wall portion of a patient's organ, and the stimulation device of the unit comprises a plurality of stimulation elements provided on and distributed along the contact surfaces. When the control device controls the stimulation device to stimulate the wall portion, the stimulation elements stimulate different areas of the wall portion along the length of the wall portion. The stimulation elements preferably comprise electric elements, as described above, for stimulating the wall portion with electric pulses. However, in most applications of the present invention, other kinds of stimulations, such as thermal stimulation, could be suitable to employ.

The operation device operates the adjustable constriction device of the constriction/stimulation unit in a manner that depends on the design of the constriction device, as will be explained by the following examples of embodiments.
1) The constriction device comprises at least two elongated clamping elements having the contact surfaces and extending along the wall portion on different sides of the organ, and the operation device operates the clamping elements to clamp the wall portion between the clamping elements to constrict the wall portion of the organ.
2) The constriction device comprises one elongate clamping element having the contact surfaces and extending along the wall portion on one side of the organ, and the operation device operates the clamping element to clamp the wall portion between the clamping element and the bone or tissue of the patient to constrict the wall portion.
3) The constriction device comprises at least two engagement elements having the contact surfaces and positioned on different sides of the organ, and the operation device rotates the engagement elements, such that the engagement elements engage and constrict the wall portion of the organ.
4) The constriction device comprises at least two articulated clamping elements having the contact surfaces and positioned on different sides of the organ, and the operation device moves the clamping elements towards each other to clamp the wall portion of the organ between the clamping elements, to constrict the wall portion.
5) The constriction device comprises at least two separate clamping elements having the contact surfaces, at least one of the clamping elements being pivoted, such that it may turn in a plane in which the loop of the constriction member extends, and the operation device turns the pivoted clamping element to change the size of the constriction opening.
6) The constriction device comprises at least one elongated constriction member having the contact surfaces, and forming means for forming the constriction member into at least a substantially closed loop around the organ, wherein the loop defines a constriction opening. The operation device operates the constriction member in the loop to change the size of the constriction opening.
   6a) The elongated constriction member comprises a belt having the contact surfaces, and the operation device operates the belt to change the longitudinal extension of the belt in the loop to change the size of the constriction opening. The forming means may form the constriction member or belt into a loop having at least one predetermined size.
   6b) The elongated constriction member is operable to change the size of the constriction opening, such that the outer circumferential confinement surface of the constriction device is changed, or, alternatively, is unchanged.
   6c) The elongated constriction member is elastic and varies in thickness as seen in a cross-section there through, and is operable to turn around the longitudinal extension of the constriction member.
   6d) The elongated constriction member comprises two substantially or partly semi-circular frame elements having the contact surfaces and hinged together, such that the semi-circular elements are swingable relative to each other from a fully open state in which they substantially or partly form a circle to a fully folded state in which they substantially form a semi-circle.
7) The constriction device is adapted to bend the wall portion of the organ to constrict the latter.

In the above noted embodiments (1) to (7), it is important that the constriction device is designed to constrict said length of the tissue wall portion of the patient's organ. For this purpose, the constriction device may include two or more of the described constriction elements/members to be applied in a row along said length of the wall portion, wherein said row extends in the direction of flow in the oviduct of the organ. Preferably, such constriction elements/members are non-inflatable and mechanically operable or adjustable.

In the above noted embodiments (1) to (7), the operation device may either mechanically or hydraulically adjust the constriction device of the constriction/stimulation unit. Also, the operation device may comprise an electrically powered operation device for operating the constriction device. For many applications of the present invention, the operation device suitably operates the constriction device, such that the through-flow area of the oviduct assumes a size in the constricted state that enables the stimulation device to contract the wall portion such that the flow in the oviduct is stopped.

### Mechanical operation

Where the operation device mechanically operates the constriction device of the constriction/stimulation unit, it may be non-inflatable. Furthermore, the operation device may comprise a servo device, which may include a gearbox. The term "servo device" encompasses the normal definition of a servo mechanism, i.e., an automatic device that controls large amounts of power by means of very small amounts of power, but may alternatively or additionally encompass the definition of a mechanism that transfers a weak force acting on a moving element having a long stroke into a strong force acting on another moving element having a short stroke. Preferably, the operation device operates the constriction device in a non-magnetic and/or non-manual manner. A motor may be operatively connected to the operation device. The operation device may be operable to perform at least one reversible function and the motor may be capable of reversing the function.

### Hydraulic Operation

Where the operation device hydraulically operates the constriction device of the constriction/stimulation unit, it includes hydraulic means for adjusting the constriction device.

In an embodiment of the invention, the hydraulic means comprises a reservoir and an expandable/contractible cavity in the constriction device, wherein the operation device distributes hydraulic fluid from the reservoir to expand the cavity, and distributes hydraulic fluid from the cavity to the reservoir to contract the cavity. The cavity may be defined by a balloon of the constriction device that abuts the tissue wall portion of the patient's organ, so that the patient's wall portion is constricted upon expansion of the cavity and released upon contraction of the cavity.

Alternatively, the cavity may be defined by a bellows that displaces a relatively large contraction element of the constriction device, for example a large balloon that abuts the wall portion, so that the patient's wall portion is constricted upon contraction of the bellows and released upon expansion of the bellows. Thus, a relatively small addition of hydraulic fluid to the bellows causes a relatively large increase in the constriction of the wall portion. Such a bellows may also be replaced by a suitably designed piston/cylinder mechanism.

Where the hydraulic means comprises a cavity in the constriction device, the device of the invention can be designed in accordance with the options listed below.
1) The reservoir comprises first and second wall portions, and the operation device displaces the first and second wall portions relative to each other to change the volume of the reservoir, such that fluid is distributed from the reservoir to the cavity, or from the cavity to the reservoir.
   1a) The first and second wall portions of the reservoir are displaceable relative to each other by at least one of a magnetic device, a hydraulic device or an electric control device.
2) The operation device comprises a pump for pumping fluid between the reservoir and the cavity.
   2a) The pump comprises a first activation member for activating the pump to pump fluid from the reservoir to the cavity and a second activation member for activating the pump to pump fluid from the cavity to the reservoir.
      2a1) The first and second activation members are operable by manual manipulation thereof.
      2a2) At least one of the activation members operates when subjected to an external predetermined pressure.
      2a3) At least one of the first and second activating members is operable by magnetic means, hydraulic means, or electric control means.
   2b) The device comprises a fluid conduit between the pump and the cavity, wherein the reservoir forms part of the conduit. The conduit and pump are devoid of any non-return valve. The reservoir forms a fluid chamber with a variable volume, and the pump distributes fluid from the chamber to the cavity by a reduction in the volume of the chamber and withdraws fluid from the cavity by an expansion of the volume of the chamber. The device further comprises a motor for driving the pump, wherein the pump comprises a movable wall of the reservoir for changing the volume of the chamber.

In all of the above noted embodiments 1 to 2b where the hydraulic means comprises an expandable cavity in the constriction device, the cavity can be exchanged by a cylinder/piston mechanism for adjusting the constriction device. In this case, the operation device distributes hydraulic fluid between the reservoir and the cylinder/piston mechanism to adjust the constriction device.

In a special embodiment of the invention, the operation device comprises a reverse servo operatively connected to the hydraulic means. The term "reverse servo" is to be understood as a mechanism that transfers a strong force acting on a moving element having a short stroke into a weak force acting on another moving element having a long stroke; i.e., the reverse function of a normal servo mechanism. Thus, minor changes in the amount of fluid in a smaller reservoir could be transferred by the reverse servo into major changes in the amount of fluid in a larger reservoir. The reverse servo is particularly suited for manual operation thereof.

Preferably, the reverse servo comprises an expandable servo reservoir containing servo fluid and a fluid supply reservoir hydraulically connected to the servo reservoir to form a closed conduit device for the servo fluid. The expandable servo reservoir has first and second wall portions, which are displaceable relative to each other in response to a change in the volume of the expandable servo reservoir.

In accordance with a first alternative, the first and second wall portions of the servo reservoir are operatively connected to the hydraulic means. The reverse servo distributes fluid between the fluid supply reservoir and the expandable servo reservoir to change the volume of the servo reservoir, whereby the hydraulic means is operated to adjust the constriction device.

In accordance with a second alternative, there is provided an implantable main reservoir containing a predetermined amount of hydraulic fluid, wherein the reverse servo is operable to distribute hydraulic fluid between the main reservoir and the hydraulic means to adjust the constriction device. More specifically, the main reservoir is provided with first and second wall portions operatively connected to the first and second wall portions of the expandable servo reservoir, such that the volume of the main reservoir is changed when the volume of the expandable servo reservoir is changed. Thus, when the reverse servo distributes servo fluid between the fluid supply reservoir and the expandable servo reservoir to change the volume of the main reservoir, hydraulic fluid is distributed from the main reservoir to the hydraulic means, or from the hydraulic means to the main reservoir. Advantageously, the servo and main reservoirs are dimensioned, such that when the volume of the servo reservoir is changed by a relatively small amount of servo fluid, the volume of the main reservoir is changed by a relatively large amount of hydraulic fluid.

In both of the above-described alternatives, the fluid supply reservoir may have first and second wall portions, which are displaceable relative to each other to change the volume of the fluid supply reservoir to distribute servo fluid between the fluid supply reservoir and the expandable servo reservoir. The first and second wall portions of the fluid supply reservoir may be displaceable relative to each other by manual manipulation, a magnetic device, a hydraulic device, or an electric control device to change the volume of the fluid supply reservoir to distribute servo fluid between the fluid supply reservoir and the expandable servo reservoir.

In all of the above noted embodiments 1 to 2b where the hydraulic means comprises an expandable cavity in the constriction device, or in embodiments where the hydraulic means comprises a hydraulically operable mechanical construction, the operation device may include the reverse servo described above. In a further embodiment of the invention, the hydraulic means include first and second hydraulically interconnected expandable/contractible reservoirs. The first reservoir is operatively connected to the constriction device, such that the constriction device changes the constriction of the patient's wall portion upon expansion or contraction of the first reservoir. By changing the volume of the second reservoir hydraulic fluid is distributed between the two reservoirs, so that the first reservoir is either expanded or contracted. This embodiment requires no non-return valve in the fluid communication conduits between the two reservoirs, which is beneficial to long-term operation of the hydraulic means.

Alternatively, the hydraulic means may include first and second hydraulically interconnected piston/cylinder mechanisms instead of the first and second reservoirs described above. The first piston/cylinder mechanism is operatively connected to the constriction device, such that the constriction device changes the constriction of the patient's wall portion upon operation of the first piston/cylinder mechanism. By operating the second piston/cylinder mechanism hydraulic fluid is distributed between the two piston/cylinder mechanisms, so that the first piston/cylinder mechanism adjusts the constriction device.

Where the constriction device does not include an expandable/contractible cavity, the constriction device may comprise at least two elongated clamping elements having the above-mentioned contact surfaces and extending along the wall portion on different sides of the organ. The hydraulic means, which may include the reverse servo described above, hydraulically moves the elongated clamping elements towards the wall portion to constrict the wall portion. For example, the constriction device may have hydraulic chambers in which the clamping elements slide back and forth, and the hydraulic means may also include a pump and an implantable reservoir containing hydraulic fluid. The pump distributes hydraulic fluid from the reservoir to the chambers to move the clamping elements against the wall portion, and distributes hydraulic fluid from the chambers to the reservoir to move the clamping elements away from the wall portion.

### More than one restriction area

The restriction device independent of which type or combination of types preferable comprises a more than one restriction area being adapted to change the restriction area over time. This will prevent any damage to the oviduct still keeping the oviduct closed avoiding any egg to pas down to the uterus thus avoiding pregnancy.

In one embodiment the system comprising a hydraulic restriction device with two or more restriction areas connected individually to two or more reservoirs with hydraulic fluid, said reservoirs adapted to be regulated to move fluid from said reservoirs individually to each of the connected restriction areas. It is possible to use only one reservoir if valves instead control to which restriction the hydraulic fluid is acting on.

The hydraulic restriction areas is adapted to be restricted for a predetermined time period preferable with some overlap in time and adapted to first restrict the restriction area closest to the ovary then changing restriction area towards the uterus. This way the restriction may all the time be kept but without risking to have any egg to pas when changing restriction area both because of the overlap in restriction and also because restriction is started first closest to the ovary where the egg is released.

The hydraulic restriction areas may be adapted to be regulated by manual manipulation thereof.

The system may also be adapted to have the changes of the restriction area cause a peristaltic wave like restriction wave in the direction towards the ovary to prevent the egg being transported down to the uterus. This peristaltic wave may be caused by any of the different types of restriction devices or combinations described earlier. This wave could prevent the egg being transported down to the uterus also without fully restricting the oviduct. This would then allow the sperm to reach the egg during the accumulation period of a few days, thus increasing likelihood of pregnancy even further.

The restriction device may also be adapted to effect a transport of the at least one egg to the uterus upon release of the oviduct. This may be with a peristaltic wave like restriction wave in the opposite direction towards the uterus.

A system of increasing likelihood of pregnancy may be adapted to restrict a first part or area of an oviduct of the patient to provide a restriction to accumulate at least one egg released from the ovary in the oviduct for a predetermined period of time, and adapted to restrict a second part of the oviduct and thereafter release the restriction of the first part and also later release the restriction of the second part, thereby allow transport of the egg down to the uterus or the system may further be adapted to also restrict a third part of the oviduct and release the restriction of the second part also it may further be adapted to release the restriction of the third part thereby allow transport of the egg down to the uterus or the system may be adapted to restrict a fourth part of the oviduct
and release the restriction of the third part. Finally it may be further adapted to release the restriction of the fourth part, thereby allow transport of the egg down to the uterus.

The number of restriction areas has no limit except practical size issues and the restriction time period is preferable divided between the restriction areas. Either the restriction area is moved from the ovary and further down towards the uterus step by step thus avoiding any interference with any accumulated egg being involved in any restriction area if a certain overlap in time is fulfilled between the consecutive restriction areas in use or the restriction areas are moved both up- and down-stream then preferable using a movement device to move the egg in the oviduct to avoid any egg being squeezed in any restriction area when restriction are moved towards the ovary or having the peristaltic like wave moving towards the ovary keeping the egg accumulated in the oviduct without fully restricting the same.

Preferable a hydraulic restriction device only partly restricting the oviduct is used maybe in combination with a stimulation device to cause the peristaltic wave in the oviduct. With the stimulation device it is then possible to move the restriction area. The hydraulic device may for example have the movement function to cause the movements necessary.

Any combination is possible of mechanical, hydraulic or stimulation device or separate use of the same.

In summary preferable more than two restricting areas is used and varying the restricting area while at least one restricting area is closed when the device being in restriction mode.

If the device is adapted to restrict the first part of the oviduct closest to the ovary it will allow the second restriction being restricted without interfering with any accumulated egg.

There are no limit how many areas could be restricted except practical and size issues.

Moving in restriction from the ovary and down towards the uterus is then the preferred way of doing it, for example three restrictions could share a time period of three days with one day each to not risking to in any way damage the oviduct,

A system adapted to restrict the restriction areas in consecutive order starting with the restriction area, part of the oviduct, closest to the ovary and thereafter restrict any new area one step closer to the uterus and further adapted to overlap in time the restriction of more than one restriction area will allow to restrict without interfering with any accumulated egg.

### PERISTALTIC LIKE WAVE OVIDUCT WALL MOVEMENTS

The restriction device may be adapted to only partly restrict the oviduct and to create peristaltic wave like movements of a part of the oviduct(s) wall to prevent the transport of an egg in an oviduct to the uterus of the human or mammal patient, and therefore accumulate at least one egg released from the ovary in the oviduct for a predetermined period of time, and wherein the sperm is able to reach the egg during the time the egg is accumulated because the oviduct is only partly restricted, and the restriction device further adapted to release the egg, controlled from outside the body, to allow the at least on egg in the oviduct a transport to the uterus.

The device is then preferable adapted to comprise at least one restricted area adapted to at least partly restrict and change the restriction area over time most likely adapted to comprise two or more restricted areas adapted to at least partly restrict different portions of the oviduct and change the restriction area over time.

The change of the restriction area may be adapted to cause a peristaltic wave like wave in the direction towards the ovary to prevent the egg being transported down to the uterus.

The restriction device may comprise a hydraulic device or a mechanical device or a stimulation device adapted to cause said peristaltic wave like restriction wave.

The device is adapted to allow a transport of the at least one egg to the uterus upon release of the oviduct and may even cause a another peristaltic wave like restriction wave in the opposite direction towards the uterus.

The device is adapted to be adjusted which could be done by manual manipulation or electrical or magnetic power.

The restriction areas is only partly restricted to allow the sperm to reach the egg and the device adapted to prevent the egg to temporary reach the uterus with the peristaltic wave, to accumulate the egg temporary in the oviduct to allow increased likelihood for pregnancy to prolong the time period the egg being reachable by the sperm.

When the restriction areas is completely restricted to prevent the egg to temporary reach the uterus, the timing of the release of the egg is crucial after having accumulated the egg temporary in the oviduct the whole procedure to allow increased likelihood for pregnancy when released.

The restriction device is adapted to and intended to repeatedly every month repeat the postoperational and non-invasive regulation of the device actively reversible to prevent transport of an egg from the ovary to the uterus in the oviduct of the patient and reverse the function by allowing the normal transport of the egg from the ovary to the uterus until pregnancy has been achieved.

The device may comprise a mechanical device or a hydraulic device or a stimulation device adapted to cause said peristaltic like wave towards the ovary.

### Restriction embodiments

The restriction device may comprises a mechanical restriction device or a hydraulic restriction device or a stimulation device or a stimulation device in combination with a mechanical or hydraulic restriction device or any other combination.

The method may include a hydraulic restriction device comprising a reservoir, for moving gas or fluid to or from said restriction device and wherein said reservoir is placed subcutaneously for
i. being reached by the patients hand for
ii. moving fluid manually to or from said restriction device Preferable two or more reservoirs could be used for restricting different areas, starting with the area closest to the ovary.

### Method movement device

The movement device is adapted to move the egg out of a varied upcoming new restricted area before a new area is restricted useful if the restriction need to be moved also in the direction towards the ovary in which case the egg could slip through if being squeezed by one restriction area and later released.

The movement device may be the same device as the restriction device, adapted to work differently when restricting or causing movements of the egg or it may be a different device as the restriction device adapted to work differently when restricting or causing movements of the egg.

The movement device may cause vibration or wave like movements in the oviduct wall thereby causing movements of the egg.

### FURTHER METHODS

A method of promoting pregnancy of a female mammal or human patient, may comprise the following steps:
restricting an oviduct of the patient postoperatively to provide a restriction to accumulate at least one egg released from the ovary in the oviduct for a predetermined period of time, and
releasing the restriction to admit any egg in the oviduct a transport to the uterus
controlling the restricting and releasing procedures from outside the patients body.

In this and other methods the predetermined period of time is adapted to avoid pregnancy and may be between 0 and 2 days or preferable 8-12 hours.

A method for placing and controlling two implanted restriction devices promoting pregnancy in a human or mammal patient, the method comprising the steps of:
- inserting a needle like tube into the abdomen of the patients body,
- using the tube like needle like to fill the abdomen with gas thereby expanding the abdominal cavity,
- placing at least two laparoscopic trocars in the patient's body,
- inserting a camera through one of the trocars into the abdomen,
- inserting at least one dissecting tool through a trocar and dissecting an area of at least one portion of the two oviducts of the patient,
- placing two implanted restriction devices, on each of the two oviducts
- adjusting the restriction devices after the operation at a time convenient to get pregnat thus,
- controlling the adjustment from outside the patients body and
- post-operatively restricting and releasing the two oviducts to increase the likelihood of getting pregnant.

Both methods above could when restricting the oviduct comprising the following steps:
- restricting a first part or area of an oviduct of the patient to provide a restriction to accumulate at least one egg released from the ovary in the oviduct for a predetermined period of time, and
- restricting a second part of the oviduct
- releasing the restriction of the first part
- allowing the oviduct shorter restriction periods at each restriction area.

May also comprise the steps of:
- releasing the restriction of the second part
- allowing transport of the egg down to the uterus

May also comprise the steps of:
- restricting a third part of the oviduct
- releasing the restriction of the second part
- allowing the oviduct shorter restriction periods at each restriction area.

May also comprise the steps of:
- releasing the restriction of the third part
- allowing transport of the egg down to the uterus

May also comprise the steps of:
- restricting a fourth part of the oviduct
- releasing the restriction of the third part
- allowing the oviduct shorter restriction periods at each restriction area.

May also comprise the steps of:
- releasing the restriction of the fourth part
- allowing transport of the egg down to the uterus In both these methods normally if the restricted first part of the oviduct being closer to the ovary this is,
- allowing the second restriction being restricted without interfering with any accumulated egg.

Normally these methods,
- having more than two restricting areas and
- varying the restricting area while
- keeping at least one restricting area closed when restriction being wished

In a preferred embodiment;
the restriction areas being restricted in consecutive order starting with the restriction area, part of the oviduct, closest to the ovary thereafter
- restricting any new area one step closer to the uterus
- overlapping in time the restriction of more than one restriction area thereby,
- restricting without interfering with any accumulated egg

A method may of course only include independent of order varying the restriction area postoperatively to allow the oviduct to recover or avoid any damage from the restriction while
- keeping the oviduct restricted.

Another method of promoting pregnancy of a female patient, comprising the following steps:
preventing the transport of an egg in an oviduct to the uterus of the human or mammal patient,
accumulating at least one egg released from the ovary in the oviduct for a predetermined period of time, by
   - casing a peristaltic like restriction wave movement of a part of the oviduct(s) wall preventing the egg being transported down to the uterus while only partly restricting the oviduct, and
   - allowing the spermie to reach the egg during the time the egg is accumulated, and
   - releasing the egg post-operatively controlled from the outside the human body to admit the at least on egg in the oviduct to allow a transport of the at least one egg to the uterus.

A method for placing the device above and controlling the implanted device promoting pregnancy in a human or mammal patient, the method comprising the steps of:
- inserting a tube like needle into the abdomen of the patients body,
- using the tube like needle like to fill the abdomen with gas thereby expanding the abdominal cavity,
- placing at least two laparoscopic trocars in the patient's body,
- inserting a camera through one of the trocars into the abdomen,
- inserting at least one dissecting tool through a trocar and dissecting an area of at least one portion of the two oviducts of the patient,
- placing two parts of the implanted device, one each on the two oviducts
- finishing the operation and withdrawing the instruments after eventual suturing and thereafter postoperatively:
- adjusting the device after the operation at a time relevant to get pregnat,
- controlling the adjustment from outside the patients body and thereby
- casing a peristaltic like wave movement of a part of the oviduct(s) wall towards the ovary preventing the egg being transported down to the uterus without fully restricting the oviduct, thereby
- preventing flow of any egg to reach the uterus in the two oviducts for a predetermined period of time and thereby
- accumulating any egg released from the ovary in the oviduct(s) by
- allowing the spermie to reach the egg during the time the egg is accumulated
- releasing any egg in the oviduct from outside the body to allow the egg to in a normal way be transported down to the uterus.

### Movement device method

Another method of preventing pregnancy of a female human or mammal patient may be provided, comprising the following steps:
- restricting a first part of an oviduct of the patient to provide a restriction to accumulate at least one egg released from the ovary in the oviduct for a predetermined period of time, and
- moving the accumulated egg by said movement device away in the oviduct towards the ovary from the restriction area,
- allowing a second part of the oviduct closer to the ovary being restricted without interfering with any accumulated egg
- releasing the restriction of the first part
- repeating the restriction of the first part
- releasing the restriction of the second part
- allowing the oviduct to recover between restriction intervals.

Preferably followed by;
- repeating moving any accumulated egg by said movement device away in the oviduct towards the ovary from the restricted first area, repeating
- allowing a second part of the oviduct closer to the ovary being restricted without interfering with any accumulated egg, further repeating
- releasing the restriction of the first part
- repeating the complete procedure allowing the oviduct to recover between restriction intervals.

Preferable also the restriction areas is adapted to be varied between three or more areas always keeping the oviduct closed.

Said movement device may comprise a vibrating device, for causing a vibration of at least a part of the wall of said oviduct causing movement of any accumulated egg, said movement being repeated.

Said movement device may comprise a mechanical device or a hydraulic device or a stimulation device or a combined device.

### Design of control device

The control device suitably controls the constriction and /or stimulation unit from outside the patient's body. Preferably, the control device is operable by the patient. For example, the control device may comprise a manually operable switch for switching on and off the constriction/stimulation unit, wherein the switch is adapted for subcutaneous implantation in the patient to be manually or magnetically operated from outside the patient's body. Alternatively, the control device may comprise a hand-held wireless remote control, which is conveniently operable by the patient to switch on and off the constriction/stimulation unit. The wireless remote control may also be designed for application on the patient's body like a wristwatch. Such a wristwatch type of remote control may emit a control signal that follows the patient's body to implanted signal responsive means of the device.

Where the control device wirelessly controls the constriction/stimulation unit from outside the patient's body, the wireless control function is preferably performed in a non-magnetic manner, i.e., the control device controls the constriction device of the constriction/stimulation unit in a non-magnetic manner. The patient may use the remote control to control the constriction/stimulation unit to adjust the stimulation intensity and/or adjust the constriction of the wall portion. The wireless remote control may comprise at least one external signal transmitter or transceiver and at least one internal signal receiver or transceiver implantable in the patient.

The wireless remote control preferably transmits at least one wireless control signal for controlling the constriction/stimulation unit. The control signal may comprise a frequency, amplitude, phase modulated signal or a combination thereof, and may be an analogue or a digital signal, or a combination of an analogue and digital signal. The remote control may transmit an electromagnetic carrier wave signal for carrying the digital or analogue control signal. Also the carrier signal may comprise digital, analogue or a combination of digital and analogue signals.

Any of the above control signals may comprise wave signals, for example a sound wave signal, an ultrasound wave signal, an electromagnetic wave signal, an infrared light signal, a visible light signal, an ultra violet light signal, a laser light signal, a microwave signal, a radio wave signal, an x-ray radiation signal or a gamma radiation signal. Alternatively, the control signal may comprise an electric or magnetic field, or a combined electric and magnetic field.

As mentioned above, the control signal may follow the patient's body to implanted signal responsive means of the device.

The control device may include a programmable internal control unit, such as a microprocessor, implantable in the patient for controlling the constriction/stimulation unit. The control device may further include an external control unit intended to be outside the patient's body, wherein the internal control unit is programmable by the external control unit. For example, the internal control unit may be programmable for controlling the constriction/stimulation unit over time, suitably in accordance with an activity schedule program. The device of the invention may comprise an external data communicator and an implantable internal data communicator communicating with the external data communicator, wherein the internal communicator feeds data related to the constriction/stimulation unit back to the external data communicator or the external data communicator feeds data to the internal data communicator.

### Source of Energy

The present invention also presents a solution for supplying energy for use in connection with the operation of the constriction/stimulation unit. Thus, in a broad sense, the present invention provides an device for controlling a flow of egg in a oviduct formed by a tissue wall of a patient's organ, wherein the device comprises an implantable constriction device for gently constricting a portion of the tissue wall to influence the flow in the oviduct, a stimulation device for intermittently and individually stimulating different areas of the wall portion, as the constriction device constricts the wall portion, to cause contraction of the wall portion to further influence the flow in the oviduct, wherein the constriction and stimulation devices form an operable constriction/stimulation unit, a source of energy, and a control device operable from outside the patient's body to control the source of energy to release energy for use in connection with the operation of the constriction/stimulation unit. In a simple form of the invention, the source of energy, such as a battery or accumulator, is implantable in the patient's body.

### Transmission of Wireless Energy

In a more sophisticated form of the invention, which is preferable, the source of energy is external to the patient's body and the control device controls the external source of energy to release wireless energy. In this sophisticated form of the invention, the device comprises an energy-transmission device that transmits the released wireless energy from outside the patient's body to inside the patient's body. Among many things the wireless energy may comprise electromagnetic energy, an electric field, an electromagnetic field or a magnetic field, or a combination thereof, or electromagnetic waves. The energy-transmission device may transmit wireless energy for direct use in connection with the operation of the constriction/stimulation unit, as the wireless energy is being transmitted. For example, where an electric motor or pump operates the constriction device, wireless energy in the form of a magnetic or an electromagnetic field may be used for direct power of the motor or pump.

Thus, the motor or pump is running directly during transmission of the wireless energy. This may be achieved in two different ways: a) using a transforming device implanted in the patient to transform the wireless energy into energy of a different form, preferably electric energy, and powering the motor or pump with the transformed energy, or b) using the wirelessly transmitted energy to directly power the motor or pump. Preferably wireless energy in the form of an electromagnetic or magnetic field is used to directly influence specific components of the motor or pump to create kinetic energy for driving the motor or pump. Such components may include coils integrated in the motor or pump, or materials influenced by magnetic fields, or permanent magnets, wherein the magnetic or electromagnetic field influences the coils to generate a current for driving the motor or pump, or influences the material or permanent magnets to create kinetic energy for driving the motor or pump. Preferably, the energy-transmission device transmits energy by at least one wireless signal, suitably a wave signal. The wave signal may comprise an electromagnetic wave signal including one of an infrared light signal, a visible light signal, an ultra violet light signal, a laser signal, a microwave signal, a radio wave signal, an x-ray radiation signal, and a gamma radiation signal. Alternatively, the wave signal may comprise a sound or ultrasound wave signal. The wireless signal may be a digital or analogue signal, or a combination of a digital and analogue signal.

### Transforming Wireless Energy

In accordance with a particular embodiment of the invention, an implantable energy-transforming device is provided for transforming wireless energy of a first form transmitted by the energy-transmission device into energy of a second form, which typically is different from the energy of the first form. The constriction/stimulation unit is operable in response to the energy of the second form. For example, the wireless energy of the first form may comprise sound waves, whereas the energy of the second form may comprise electric energy. In this case, the energy-transforming device may include a piezo-electric element for transforming the sound waves into electric energy. Optionally, one of the energy of the first form and the energy of the second form may comprise magnetic energy, kinetic energy, sound energy, chemical energy, radiant energy, electromagnetic energy, photo energy, nuclear energy or thermal energy. Preferably, one of the energy of the first form and the energy of the second form is non-magnetic, non-kinetic, non-chemical, non-sonic, non-nuclear or non-thermal.

The energy-transforming device may function differently from or similar to the energy-transmission device. In a special embodiment, the energy-transforming device comprises at least one element, such as at least one semiconductor, having a positive region and a negative region, when exposed to the energy of the first form transmitted by the energy-transmission device, wherein the element is capable of creating an energy field between the positive and negative regions, and the energy field produces the energy of the second form. More specifically, the element may comprise an electrical junction element, which is capable of inducing an electric field between the positive and negative regions when exposed to the energy of the first form transmitted by the energy-transmission device, whereby the energy of the second form comprises electric energy.

The energy-transforming device may transform the energy of the first form directly or indirectly into the energy of the second form. An implantable motor or pump for operating the constriction device of the constriction/stimulation unit may be provided, wherein the motor or pump is powered by the energy of the second form. The constriction device may be operable to perform at least one reversible function and the motor may be capable of reversing the function. For example, the control device may shift polarity of the energy of the second form to reverse the motor.

The energy-transforming device may directly power the motor or pump with the transformed energy, as the energy of the second form is being transformed from the energy of the first form. Preferably, the energy-transforming device directly operates the constriction/stimulation unit with the energy of the second form in a non-magnetic, non-thermal or non-mechanical manner.

Normally, the constriction/stimulation unit comprises electric components that are energized with electrical energy. Other implantable electric components of the device may be at least one voltage level guard or at least one constant current guard. Therefore, the energy-transforming device may transform the energy of the first form into a direct current or pulsating direct current, or a combination of a direct current and pulsating direct current. Alternatively, the energy-transforming device may transform the energy of the first form into an alternating current or a combination of a direct and alternating current.

The device of the invention may comprise an internal source of energy implantable in the patient for supplying energy for the operation of the constriction/stimulation unit. The device may further comprise an implantable switch operable to switch from an "off' mode, in which the internal source of energy is not in use, to an "on" mode, in which the internal source of energy supplies energy for the operation of the constriction/stimulation unit, and/or for energizing implanted electronic components of the device. The switch may be operable by the energy of the first form transmitted by the energy-transmission device or by the energy of the second form supplied by the energy-transforming device. The described switch arrangement reduces power consumption of the device between operations.

The internal source of energy may store the energy of the second form supplied by the energy-transforming device. In this case, the internal source of energy suitably comprises an accumulator, such as at least one capacitor or at least one rechargeable battery, or a combination of at least one capacitor and at least one rechargeable battery. Where the internal source of energy is a rechargeable battery it may be charged only at times convenient for the patient, for example when the patient is sleeping. Alternatively, the internal source of energy may supply energy for the operation of the constriction/stimulation unit but not be used for storing the energy of the second form. In this alternative, the internal source of energy may be a battery and the switch described above may or may not be provided.

Suitably, the device of the invention comprises an implantable stabilizer for stabilizing the energy of the second form. Where the energy of the second form is electric energy the stabilizer suitably comprises at least one capacitor.

The energy-transforming device may be designed for implantation subcutaneously in the abdomen, thorax or cephalic region of the patient. Alternatively, it may be designed for implantation in an orifice of the patient's body and under the mucosa or intramuscularly outside the mucosa of the orifice.

Although the constriction/stimulation unit in the embodiments described above is designed as a single piece, which is most practical for implantation, it should be noted that as an alternative the constriction device and stimulation device could be designed as separate pieces. Any one of the constriction and stimulation units described above may alternatively be replaced by two or more separate constriction/stimulation elements, which are controlled independently of one another.

Where the a device is used for controlling the flow of eggs into the uterus of a female, the device comprises an implantable constriction device for constricting each one of the female's uterine tubes to restrict the passageway thereof, and a control device for controlling said constriction device to constrict the uterine tube such that an egg appearing in the passageway of the uterine tube is prevented from entering the uterine cavity, and to release the uterine tube such that an egg existing in the passageway of the uterine tube is allowed to enter the uterine cavity. The constriction device may gently constrict at least one portion of the tissue wall of the uterine tube to restrict the passageway thereof, and an implantable stimulation device may be provided for stimulating the tissue wall portion, wherein the control device controls said stimulation device to stimulate the tissue wall portion, as said constriction device constricts the tissue wall portion, to cause contraction of the tissue wall portion to further restrict the passageway of the uterine tube.

Alternatively, the egg flow control device comprises an implantable constriction device for gently constricting at least one portion of the tissue wall of each one of the female's uterine tubes to restrict the passageway thereof, a stimulation device for stimulating the tissue wall portion of the uterine tube, and a control device for controlling said stimulation device to stimulate the tissue wall portion, as said constriction device constricts the tissue wall portion, to cause contraction of the tissue wall portion to further restrict the passageway of the uterine tube to prevent an egg existing in the uterine tube from entering the uterine cavity.

Alternatively, the egg flow control device comprises an implantable stimulation device for stimulating a portion of the tissue wall of each one of the female's uterine tubes, and a control device for controlling said stimulation device to stimulate the tissue wall portion of the uterine tube to cause contraction of the tissue wall portion, such that the passageway of the uterine tube is restricted to prevent an egg appearing in the uterine tube from entering the uterine cavity, and to cease stimulating the tissue wall portion of the uterine tube to allow an egg existing in the passageway of the uterine tube to enter the uterine cavity.

The present invention also provides a method for using an device as described above to control a flow of egg in a oviduct formed by a tissue wall of a patient's organ, the method comprising:
providing a wireless remote control adapted to control the constriction device and/or stimulation device from outside the patient's body, and
operating the wireless remote control by the patient, when the patient wants to influence the flow of egg in the oviduct.

The present invention also provides a method for controlling a flow of egg in a oviduct formed by a tissue wall of a patient's organ, the method comprising:
a) gently constricting at least one portion of the tissue wall to influence the flow in the oviduct, and
b) stimulating the constricted wall portion to cause contraction of the wall portion to further influence the flow in the oviduct.

### BRIEF DESCRIPTION OF THE DRAWINGS

Fig. 1A illustrates a device according to the invention applied on the oviducts of a female patient, wherein devices are in a non-restricting operating state.
Fig. 1B is a view similar to that of Fig. 1A, but wherein devices are in a restricting operating state.
Fig. 2A illustrates a device according to the invention with remote control applied on the oviducts of a female patient, wherein devices are in a non-restricting operating state.
Fig. 2B is a view similar to that of Fig. 2A, but wherein devices are in a restricting operating state.
Fig. 3A is a schematic view of a hydraulic operation means with a subcutaneously placed reservoir suited for operating the device of the embodiments of Fig. 1A, 1B.
Fig. 3B shows the embodiment of Fig. 3A with the constriction device constricting a tissue wall of a patient's oviduct.
Fig. 4A is a schematic view of mechanical operation means suited for operating the constriction device of the embodiments of Figs. 2-11.
Fig. 4B shows the embodiment of Fig. 4A with the constriction device constricting a tissue wall of a patient's organ.
Fig. 4C shows a modification of the embodiment of Fig. 4B.
Figs. 5A, 5B and 5C are cross-sections of the embodiment of Fig. 2 showing different states of operations with the device applied on a tissue wall of a patient's organ.
Figs. 6A, 6B and 6C are cross-sections of a modification of the embodiment of Fig. 2 showing different states of operations with the device applied on a tissue wall of a patient's organ.
Figs. 7A, 7B, 7C, 7D and 7E schematically illustrate different states of operation of a general embodiment of an device according to the present invention.
Figs. 7F, 7G and 7H illustrate different states of operation of a modification of the general embodiment.
Figs. 7I, 7K and 7L illustrate an alternative mode of operation of the modification of the general embodiment.
Fig. 8A is a pulse/time diagram showing electric stimulation pulses generated by the device of the invention for stimulating a tissue wall of a patient's organ.
Fig. 8B is pulse/time diagram showing a modification of the electric stimulation shown in Fig. 8A, in which pulses of mixed frequencies and/or amplitudes are employed.
Figs. 8C and 8D show two pulse/time diagrams, respectively, representing electric stimulation of two different areas of the tissue wall with pulses forming pulse trains.
Fig. 9A is a longitudinal cross-section of an embodiment of the device of the invention including a thermal stimulation device, wherein the device is constricting a tissue wall of a patient's oviduct.
Fig. 9B is the same embodiment of Fig. 9A with the thermal stimulation device activated.
Fig. 10A is a schematic view of a hydraulically operable inflatable constriction device for use in accordance with the invention.
Fig. 10B is the same embodiment shown in Fig. 10A with the constriction device inflated.
Figs. 11A, 11B, 11C and 11D are block diagrams illustrating four different principles for hydraulic operation of the constriction device shown in Fig. 10A.
Fig. 12 is a cross-sectional view of a reservoir having a variable volume controlled by a remote control motor.
Figs. 13A and 13B are perspective views of a reverse servo in accordance with a particular embodiment of the hydraulic operation principle shown in Fig. 11C.
Fig. 14 is a schematic view of another hydraulically operable constriction device for use in accordance with the invention.
Fig. 15A illustrates the constriction device of Fig. 34 in a constricted state.
Fig. 15B illustrates the constriction device of Fig. 14 in a released state.
Fig. 16 is a schematic block diagram illustrating a general embodiment of the device of the invention, in which energy is transferred to energy consuming components of the device implanted in the patient.
Figs. 17 to 28 are schematic block diagrams illustrating twelve embodiments, respectively, based on the general embodiment shown in Fig. 37, wherein wireless energy is transmitted from outside a patient's body to energy consuming components of the device implanted in the patient.
Fig. 29 is a block diagram illustrating control components of an embodiment of the invention.
Fig. 30 is a schematic view of exemplary circuitry of an embodiment of the invention, in which wireless energy is transformed into a current.

### DETAILED DESCRIPTION OF THE INVENTION

Referring to the drawing figures, like reference numerals designate identical or corresponding elements throughout the several figures.

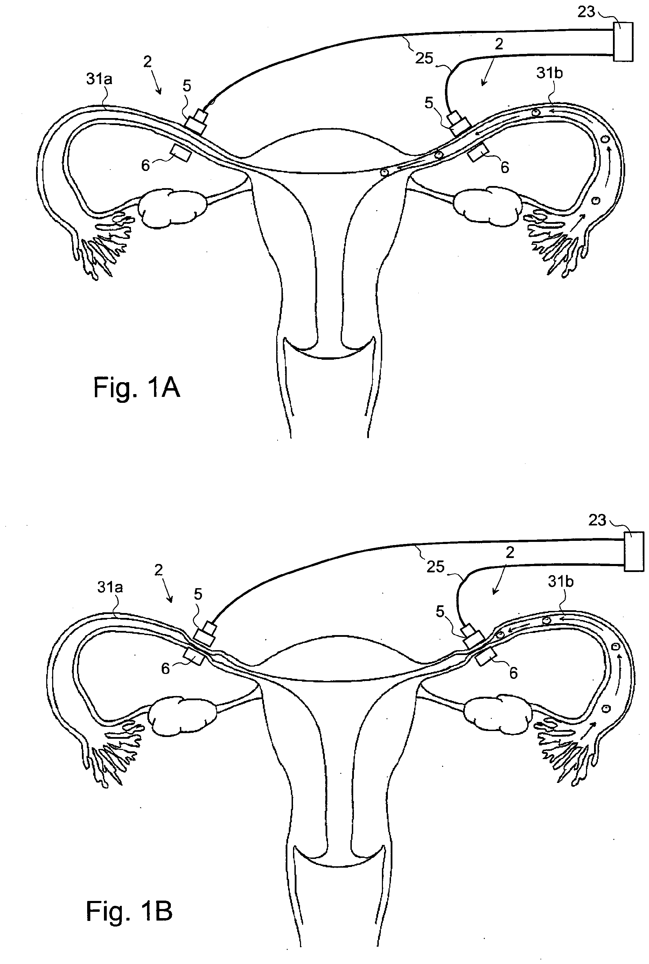

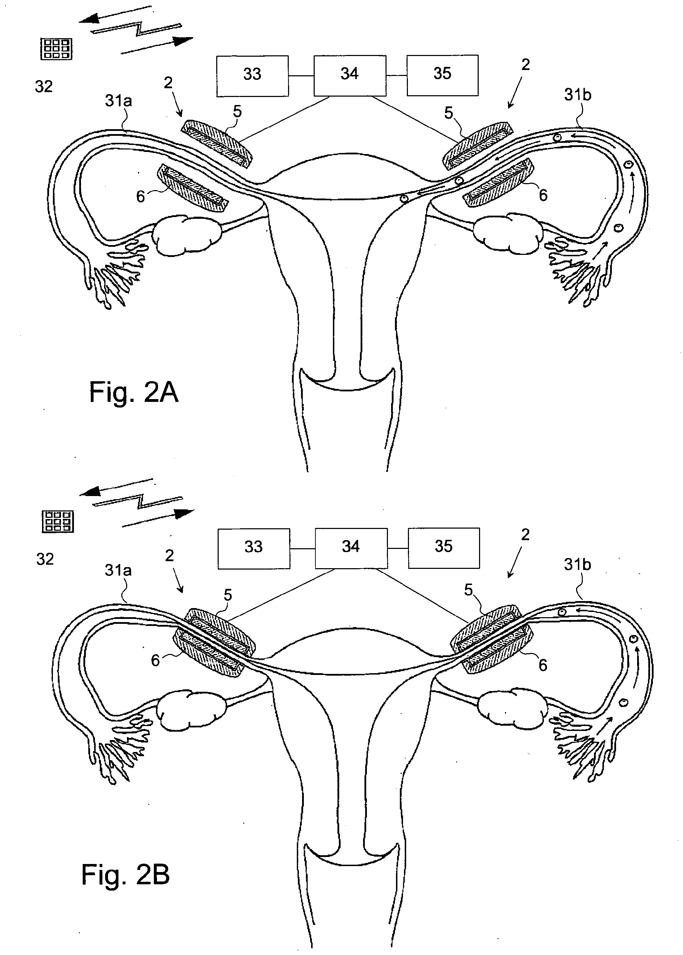

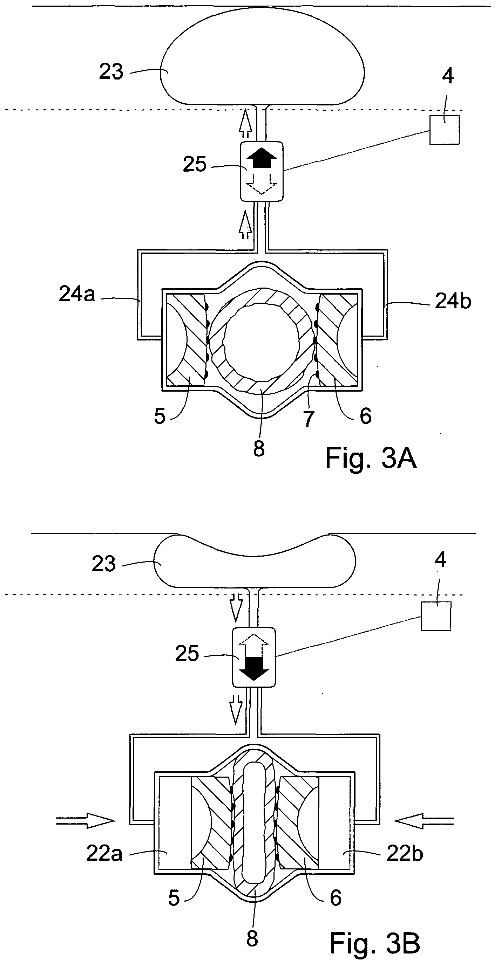

Figs. 1A and 1B illustrates a first embodiment of device for treating a female patient to promote pregnancy applied on the oviducts 31a, 31b of a female patient. Clamping elements 5, 6 of a restriction or constriction device 2 constrict the oviducts 31a, 31b. (For the sake of clarity, the housing is not shown and the clamping elements 5, 6 are exaggerated.) In this embodiment, a control device includes a subcutaneously implanted push button that is manually switched by the patient between "on" and "off'. This control device will be described in more detail below with reference to Figs. 3A AND 3B. Such a manually operable push button may also be provided in combination with a remote control as an emergency button to allow the patient to stop the operation of the device in case of emergency or malfunction. Such a remote control will be described below with reference to Figs. 2A and 2B. This device could also be fully manually controlled by manual manipulation of for example a hydraulic reservoir controlling a hydraulic device such as described in Fig. 3A-3D or 15A and 15B or 10A and 10B or any of 11A -11D. Please observe that only a reservoir moving fluid manually as in Fig. 3C and 3D may be used to adjust the device. This reservoir may be controlled in such a way that the change of the reservoir volume will be stable after the manual manipulation of the reservoir wall has taken place. In this particular case is showed a looking device but many different ways may be used, Small amount of fluid in the reservoir may also case a larger movement of the device as described in some of the embodiments above.

Figs. 2A and 2B illustrates an alternative embodiment applied on the oviducts 31a, 31b of a female patient. The clamping elements 5, 6 of the constriction device 2 constrict the oviducts 31a, 31b. (For the sake of clarity, the housing is not shown and the clamping elements 5, 6 are exaggerated.) In this embodiment, a control device includes an external control unit in the form of a hand-held wireless remote control 32, and an implanted internal control unit 33, which may include a microprocessor, for controlling the constriction and stimulation devices. The remote control 32 is operable by the patient to control the internal control unit 33 to switch on and off the device.

The internal control unit 33 controls an implanted operation device 34 to move the clamping elements 5, 6. An implanted source of energy 35, such as a rechargeable battery, powers the operation device 34. The internal control unit 33, which may be implanted subcutaneously or in the abdomen, may also work as en energy receiver, i.e., for transforming wireless energy into electric energy and charging the implanted source of energy 35 (rechargeable battery) with the electric energy.

An implanted sensor 36 senses a physical parameter of the patient, such as the temperature, wherein the internal control unit 33 controls the constriction device 2 and/or the stimulation device 3 in response to signals from the sensor 36. In this embodiment the sensor 36 is a hormone level sensor, wherein the internal control unit 33 controls the constriction device and/or stimulation device to change the constriction of the patient's oviduct 31 in response to the sensor 36 sensing a predetermined value of measured value. For example, the control unit 33 may control the constriction device and/or stimulation device to increase the constriction of the patient's oviduct 31 in response to the sensor sensing an increased or decreased hormone level. Alternatively or in combination, the remote control 32 controls the constriction device and/or a stimulation device in response to signals from the sensor 36, in the same manner as the internal control unit 33.

The remote control 32 may be equipped with means for producing an indication, such as a sound signal or displayed information, in response to signals from the sensor 36. When the patient's attention is taken by such an indication indicating a release of the oviduct based on said sensor input.. The patient may use the remote control to control the constriction device or stimulation device to pump eggs through the oviducts of the patient.

Figs. 3A and 3B show hydraulic operation means suited for operating the constriction device of the embodiments described above with reference to Figs. 1A and 1B. Specifically, Figs. 3A and 3B show the device of Figs. 1A and 1B provided with such means for hydraulic operation of the constriction device 2. Thus, the housing 1 forms two hydraulic chambers 22a and 22b, in which the two clamping elements 5, 6 are slidable back and forth relative to the tubular tissue wall portion 8 of a patient's oviduct. The hydraulic operation means include an expandable reservoir 23, such as an elastic balloon, containing hydraulic fluid, conduits 24a and 24b between the reservoir 23 and the hydraulic chambers 22a, 22b, and a two-way pump 25 for pumping the hydraulic fluid in the conduits 24a, 24b. The reservoir 23 is subcutaneously placed between the skin, shown with a solid line in the figure, and the normally fascial/muscular layer, shown with a dashed line. The control device 4 controls the pump 25 to pump hydraulic fluid from the reservoir 23 to the chambers 22a, 22b to move the clamping elements 5, 6 against the wall portion 8, whereby the tubular wall portion 8 is constricted, see Fig. 3B, and to pump hydraulic fluid from the chambers 22a, 22b to the reservoir 23 to move the clamping elements 5, 6 away from the wall portion 8, whereby the tubular wall 8 is released, see Fig. 3A.

Alternatively, the embodiment of Figs. 3A and 3B may be manually operated by applying suitable manually operable hydraulic means for distributing the hydraulic fluid between the expandable reservoir 23 and the hydraulic chambers 22a, 22b. In this case the pump 25 is omitted. Also in this case is the control device 4 only manual manipulation of the reservoir. An example is showed in Fig. 3C and 3D. In this particular embodiment is supplied a small looking of the reservoir wall (4b), but this is only one of many ways of solving this embodiment.

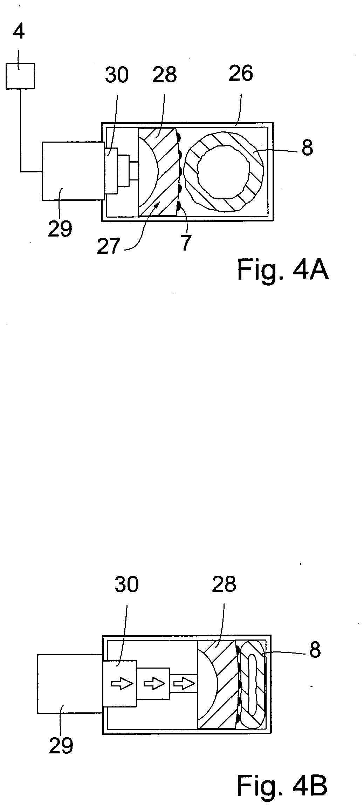

Figs. 4A and 4B schematically show a mechanically operable embodiment of the invention, comprising an open ended tubular housing 26 applied on the tubular tissue wall portion 8 of a patient's organ, a constriction device 27 arranged in the housing 26 and a control device 4 for controlling the constriction device 27. A stimulation device (not shown) as described above is also provided in the housing 26. The constriction device 27 includes a clamping element 28, which is radially movable in the tubular housing 26 towards and away from the tubular wall portion 8 between a retracted position, see Fig. 4A, and a clamping position, see Fig. 4B, in which the clamping element 28 gently constricts the tubular wall portion 8. Mechanical operation means for mechanically operating the clamping element 28 includes an electric motor 29 attached to the housing 26 and a telescopic device 30, which is driven by the motor 29 and operatively connected to the clamping element 28. The control device 4 controls the electric motor 29 to expand the telescopic device 30 to move the clamping element 28 against the wall portion 8, whereby the tubular wall portion 8 is constricted, see Fig. 4B, and controls the motor 29 to retract the telescopic device 30 to move the clamping element 28 away from the wall portion 8, whereby the wall portion 8 is released, see Fig. 4A.

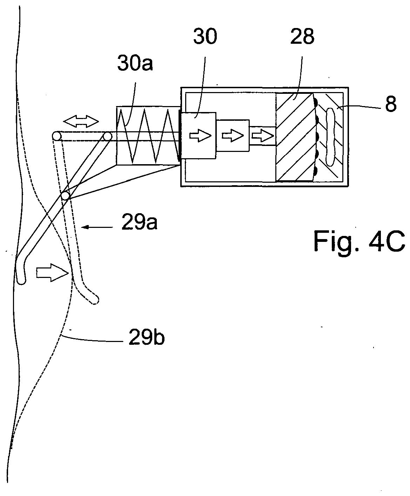

Alternatively, the motor 29 may be omitted and the telescopic device 30 be modified for manual operation, as shown in Fig. 4C. Thus, a spring 30a may be provided acting to keep the telescopic device 30 expanded to force the clamping element 28 against the wall portion 8. The mechanical operation means may include a subcutaneously implanted lever mechanism 29a that is operatively connected to the telescopic device 30. The patient may push the lever mechanism 29a through the patient's skin 29b to pull the telescopic device 30 against the action of the spring 30a to the retracted position of the telescopic device 30, as indicated in phantom lines. When the patient releases the lever mechanism 29a, the spring 30a expands the telescopic device 30, whereby clamping element 28 is forced against the wall portion 8.

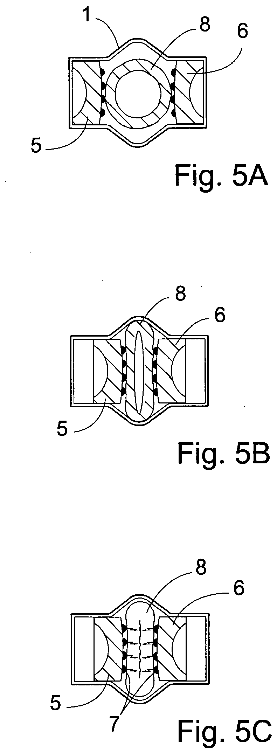

Figs. 5A - 5C illustrate in principle the function of the device of Fig. 1 when the device is applied on a portion 8 of a tubular tissue wall of a patient's oviduct. Thus, Fig. 5A shows the device in a non-clamping state, in which the clamping elements 5, 6 are in their retracted positions and the wall portion 8 extends through the open ends of the housing 1 without being constricted by the clamping elements 5, 6. Fig. 5B shows the device in a clamping state, in which the clamping elements 5, 6 have been moved from their retracted positions to their clamping positions, in which the clamping elements 5, 6 gently constrict the wall portion 8 to a constricted state, in which the blood circulation in the constricted wall portion 8 is substantially unrestricted and the flow in the oviduct of the wall portion 8 is restricted. Fig. 5C shows the device in an optional stimulation state, in which the clamping elements 5, 6 constrict the wall portion 8 and the electrical elements 7 of the stimulation device 3 electrically stimulate different areas of the wall portion 8, so that the wall portion 8 contracts (thickens) and closes the oviduct.

When the device is in its optional stimulation state, it is important to stimulate the different areas of the wall portion 8 in a manner so that they essentially maintains their natural physical properties over time to prevent the areas from being injured. Consequently, the control device 4 controls the stimulation device 3 to intermittently stimulate each area of the wall portion 8 during successive time periods, wherein each time period is short enough to maintain over time satisfactory blood circulation in the area. Furthermore, the control device 4 controls the stimulation of the areas of the wall portion 8, so that each area that currently is not stimulated restores substantially normal blood circulation before it is stimulated again. To maintain over time the effect of stimulation, i.e., to keep the oviduct closed by maintaining the wall portion 8 contracted, the control device 4 controls the stimulation device 3 to stimulate one or more of the areas at a time and to shift the stimulation from one area to another over time. The control device 4 may control the stimulation device 3 to cyclically propagate the stimulation of the areas along the tubular wall portion 8, for example, in accordance with a determined stimulation pattern. To achieve the desired reaction of the tissue wall during the stimulation thereof, the control device may control the stimulation device to, preferably cyclically, vary the intensity of the stimulation of the wall portion 8.

It will be appreciated that the fully restricted state shown in Fig. 5C can be obtained by purely mechanical means not involving electrical stimulation.

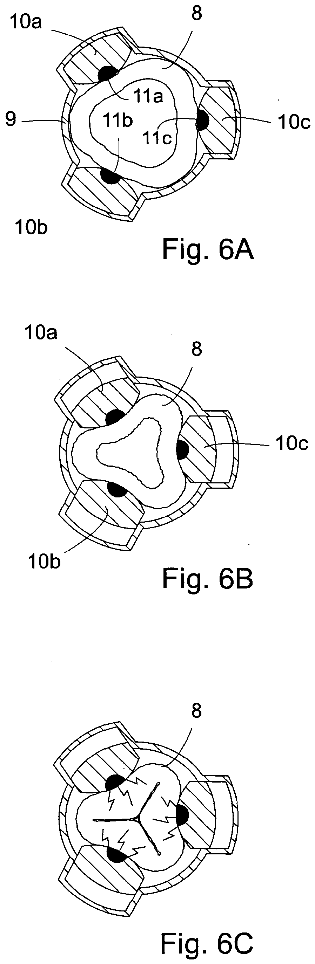

Figs. 6A - 6C show another embodiment of the invention which includes a tubular housing 9 and three elongate clamping elements 10a, 10b, 10c, which are radially movable in the tubular housing 9 towards and away from a central axis thereof between retracted positions, see Fig. 6A, and clamping positions, see Fig. 6B. The three clamping elements 10a-10c are symmetrically disposed around the central axis of the housing 9. The stimulation device of this embodiment includes electrical elements 11a, 11 b, 11c that form a series of groups of elements extending longitudinally along the elongate clamping elements 10a-10c, wherein the electrical elements 11a - 11c of each group of electrical elements form a path of three electrical elements 11a, 11b and 11c extending circumferentially around the central axis of the housing 9. The three electrical elements 11a - 11c of each group are positioned on the three clamping elements 10a-10c, respectively. Thus, the path of three electrical elements 11a-11c extends around the patient's organ. Of course, the number of electrical elements 11a-11c of each path of electrical elements can be greater than three, and several parallel rows electrical elements 11a-11c can form each path of electrical elements.

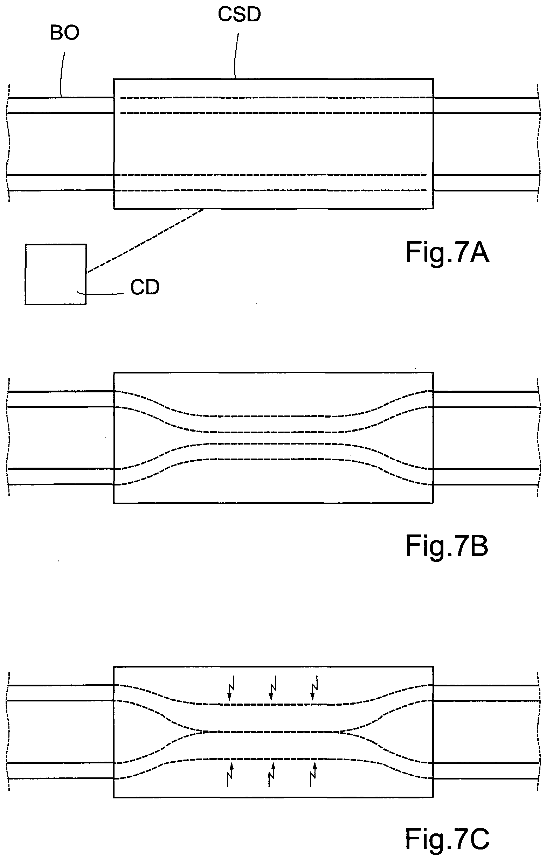

Figs. 7A, 7B and 7C schematically illustrate different states of operation of a generally designed device according to the present invention, when the device is applied on a wall portion of an oviduct designated BO. The device includes a constriction device and a stimulation device, which are designated CSD, and a control device designated CD for controlling the constriction and stimulation devices CSD. Fig. 7A shows the device in an inactivation state, in which the constriction device does not constrict the organ BO and the stimulation device does not stimulate the organ BO. Fig. 7B shows the device in a constriction state, in which the control device CD controls the constriction device to gently constrict the wall portion of the organ BO to a constricted state, in which the blood circulation in the constricted wall portion is substantially unrestricted and the flow in the oviduct of the wall portion is restricted. Fig. 7C shows the device in a stimulation state, in which the control device CD controls the stimulation device to stimulate different areas of the constricted wall portion, so that almost the entire wall portion of the organ BO contracts (thickens) and closes the oviduct.

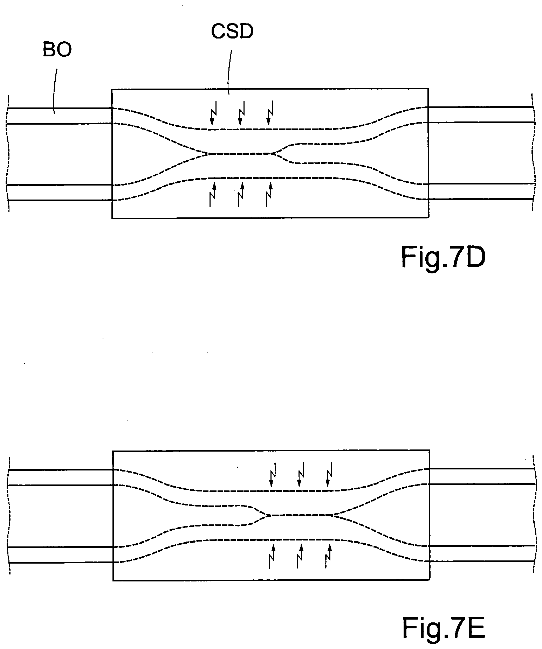

Figs. 7D and 7E show how the stimulation of the constricted wall portion can be cyclically varied between a first stimulation mode, in which the left area of the wall portion (see Fig. 7D) is stimulated, while the right area of the wall portion is not stimulated, and a second stimulation mode, in which the right area of the wall portion (see Fig. 7E) is stimulated, while the left area of the wall portion is not stimulated, in order to maintain over time satisfactory blood circulation in the constricted wall portion.

It should be noted that the stimulation modes shown in Figs. 7D and 7E only constitute a principle example of how the constricted wall portion of the organ BO may be stimulated. Thus, more than two different areas of the constricted wall portion may be simultaneously stimulated in cycles or successively stimulated. Also, groups of different areas of the constricted wall portion may be successively stimulated.

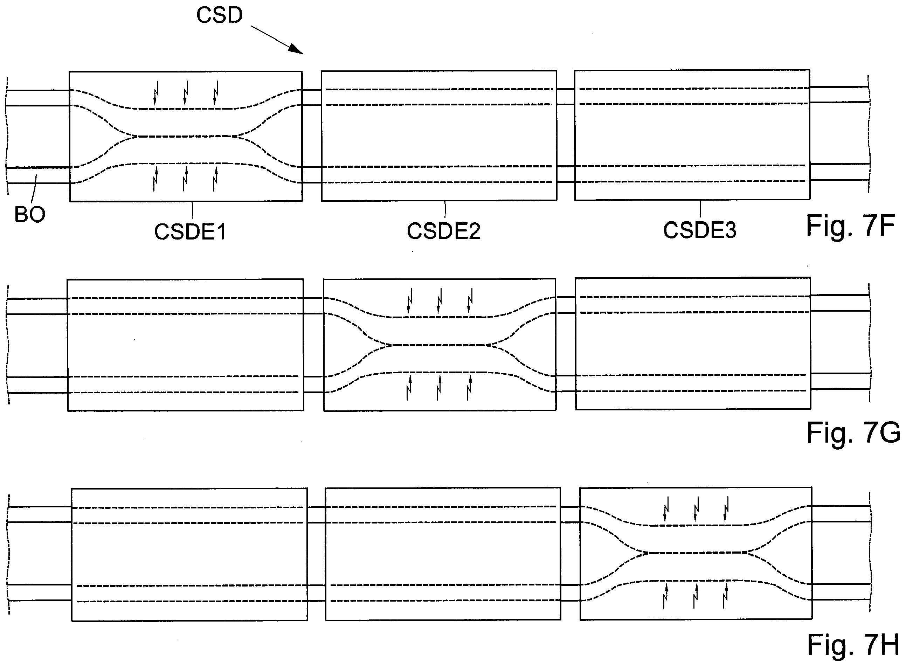

Figs. 7F, 7G and 7H illustrate different states of operation of a modification of the general embodiment shown in Figs. 7A-7E, wherein the constriction and stimulation devices CSD include several separate constriction/stimulation elements, here three elements CSDE1, CSDE2 and CSDE3. Fig. 7F shows how the element CSDE1 in a first state of operation is activated to both constrict and stimulate the organ BO, so that the oviduct of the organ BO is closed, whereas the other two elements CSDE2 and CSDE3 are inactivated. Fig. 7G shows how the element CSDE2 in a second following state of operation is activated, so that the oviduct of the organ BO is closed, whereas the other two elements CSDE1 and CSDE3 are inactivated. Fig. 7H shows how the element CSDE3 in a following third state of operation is activated, so that the oviduct of the organ BO is closed, whereas the other two elements CSDE1 and CSDE2 are inactivated. By shifting between the first, second and third states of operation, either randomly or in accordance with a predetermined sequence, different portions of the organ can by temporarily constricted and stimulated while maintaining the oviduct of the organ closed, whereby the risk of injuring the organ is minimized. It is also possible to activate the elements CSDE1-CSDE3 successively along the oviduct of the organ to move fluids and/or other bodily matter in the oviduct.

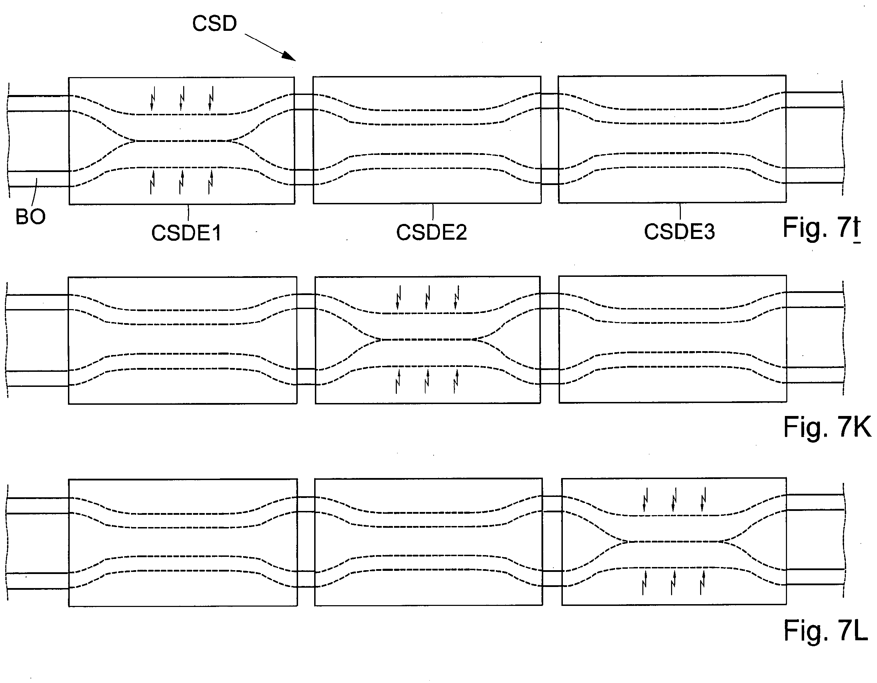

Figs. 7I, 7K and 7L illustrate an alternative mode of operation of the modification of the general embodiment. Thus, Fig. 7I shows how the element CSDE7 in a first state of operation is activated to both constrict and stimulate the organ BO, so that the oviduct of the organ BO is closed, whereas the other two elements CSDE2 and CSDE3 are activated to constrict but not stimulate the organ BO, so that the oviduct of the organ BO is not completely closed where the elements CSDE2 and CSDE3 engage the organ BO. Fig. 7K shows how the element CSDE2 in a second following state of operation is activated to both constrict and stimulate the organ BO, so that the oviduct of the organ BO is closed, whereas the other two elements CSDE7 and CSDE3 are activated to constrict but not stimulate the organ BO, so that the oviduct of the organ BO is not completely closed where the elements CSDE1 and CSDE3 engage the organ BO. Fig. 7L shows how the element CSDE3 in a following third state of operation is activated to both constrict and stimulate the organ BO, so that the oviduct of the organ BO is closed, whereas the other two elements CSDE1 and CSDE2 are activated to constrict but not stimulate the organ BO, so that the oviduct of the organ BO is not completely closed where the elements CSDE1 and CSDE2 engage the organ BO. By shifting between the first, second and third states of operation, either randomly or in accordance with a predetermined sequence, different portions of the organ can by temporarily stimulated while maintaining the oviduct of the organ closed, whereby the risk of injuring the organ is reduced. It is also possible to activate the stimulation of the elements CSDE1-CSDE3 successively along the oviduct of the organ BO to move fluids and/or other bodily matter in the oviduct.

This embodiment, with a combination of a mechanical or hydraulic partly restricting system and a stimulation system varying the stimulation position, is preferable used when one wants to close the oviduct in a safe way. The system is energy efficient because preferable only stimulation need to be changed in position and the oviduct allows to recover until the stimulation comes back to the same position again. If the restriction areas are placed closer to each other and maybe the mechanical or hydraulic restriction is continuous, a peristaltic like wave could be created in any direction in the oviduct. If the restriction is moved in consecutive order starting with the restriction closest to the ovary, no egg may be squeezed in the restriction area. If restriction are moved in the other direction towards the uterus a movement device could be used to move any egg away from any new upcoming restriction area. Such a movement device could be the hydraulic or mechanical partly restricting device causing movements in the oviduct wall before the stimulation device close the area.

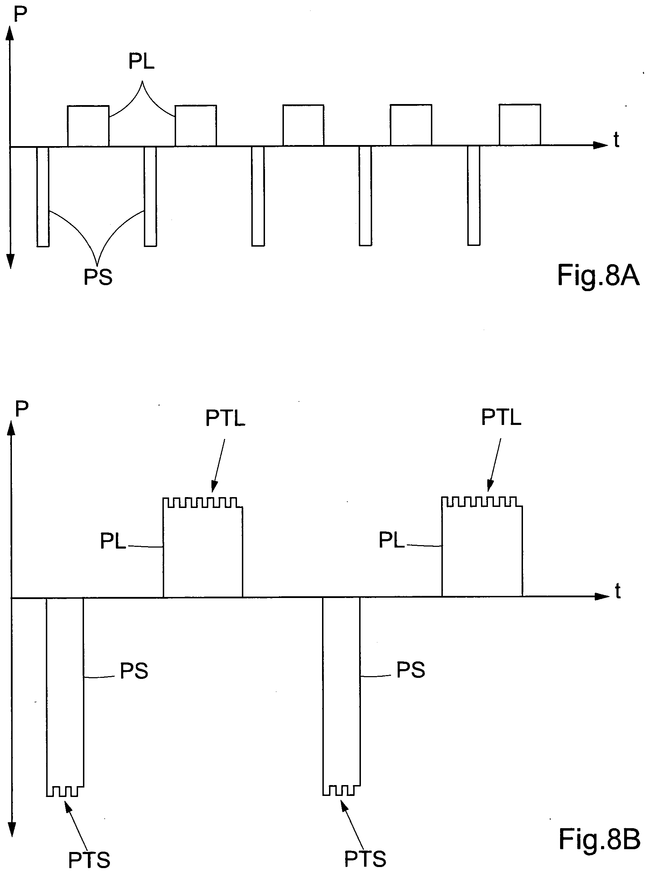

Fig. 8B is a pulse/time diagram showing a modification of the electric stimulation shown in Fig. 8A. Thus, the pulse combination of Fig. 8A is mixed with a pulse train combination having a first relatively long pulse train PTL of high frequency/low amplitude pulses, appearing simultaneously with the positive pulse PL of the pulse combination of Fig. 8A, and a second relatively short pulse train PTS of high frequency/low amplitude appearing simultaneously with the negative pulse PS of the pulse combination shown in Fig. 8A. As a result, the high frequency/low amplitudes pulse trains PTL and PTS are superimposed on the positive and negative pulses PL and PS of Fig. 8A, as illustrated in Fig. 8B. The pulse configuration of Fig. 8B, and variations thereof, is beneficial to use in connection with the stimulation of particular human organs, in order to achieve the desired stimulation effect.

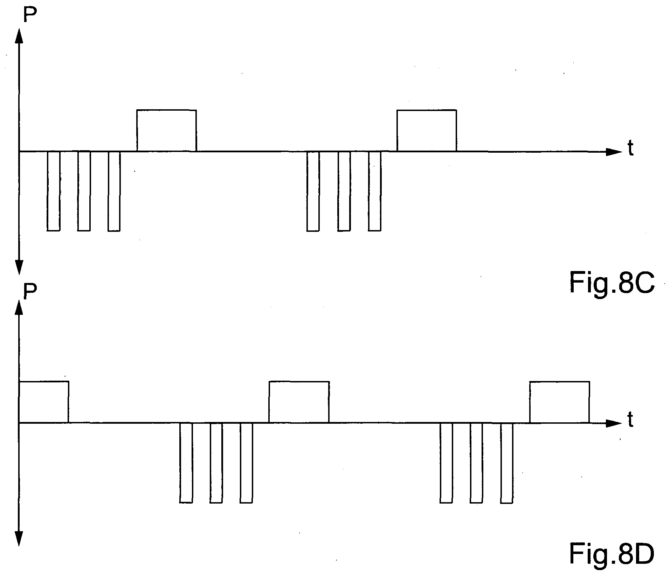

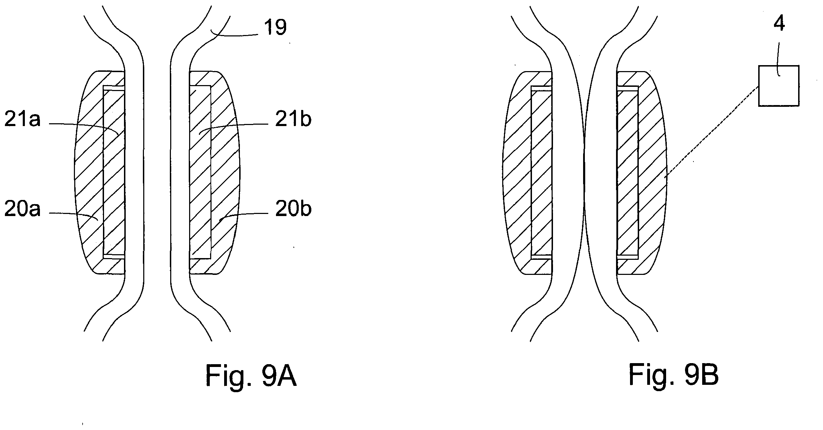

Preferably, the electric pulses form pulse trains, as illustrated in the Pulse/time diagrams P/t of Figs. 8C and 8D. The Pulse/time diagram P/t of Fig. 9A represents an individual area of the wall portion of the patient's tubular organ, which is stimulated with a pulse train 18A. The pulse train 18A includes three initial negative pulses, each of which is of short duration and high amplitude (voltage), and one positive pulse of long duration and low amplitude following the negative pulses. After a delay to enable the area of the organ to restore substantially normal blood circulation, the pulse train 18A is repeated.

The Pulse/time diagram P/t of Fig. 8D represents another individual area of the wall portion, which is stimulated with a pulse train 18B having the same configuration as the pulse train 18A. The pulse trains 18A and 18B are shifted relative to each other, so that they partially overlap one another to ensure that the constricted wall portion always is stimulated to contract as desired.

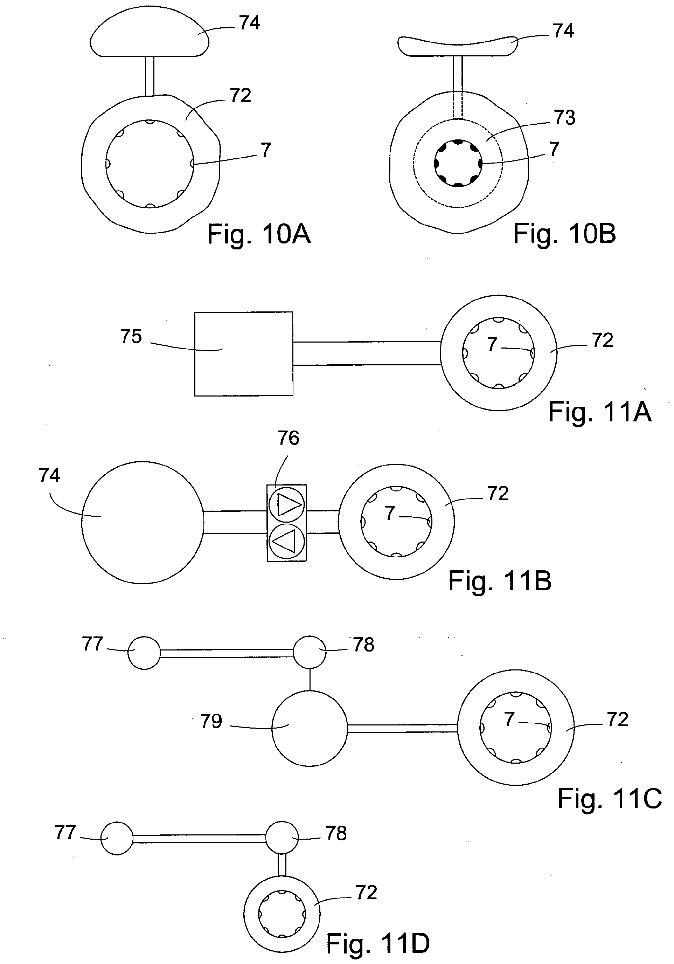

Figs. 9A and 9B show another embodiment of the invention that controls blood flow in a blood vessel 19, comprising a constriction device with two clamping elements 20a and 20b, a stimulation device in the form of two thermal stimulation elements 21a and 21b integrated in the clamping elements 20a, 20b, respectively, and a control device 4 for controlling the clamping elements 20a, 20b and stimulation elements 21a, 21b. The clamping elements 20a and 20b are movable towards and away from each other in the same manner as described above in connection with the embodiment according to Figs. 5A-5C. The thermal stimulation elements 21a and 21b, which may include Pertier elements, are positioned on the clamping elements 20a, 20b, so that the thermal elements 21a are facing the thermal elements 21b. Fig. 11A shows how the clamping elements 20a, 20b constrict the blood vessel 19, so that the blood flow is restricted. Fig. 11B shows how the control device 4 controls the thermal stimulation elements 21a, 21b to cool the wall of the blood vessel 19, so that the wall contracts and closes the blood vessel 19. To release the blood vessel 19, the control device 4 controls the thermal stimulation elements 21a, 21b to heat the wall of the blood vessel 19, so that the wall expands.

Figs. 10A and 10B show a hydraulically operable elongated constriction device in the form of a band 72 having an expandable/contractible cavity 73, which is in fluid communication with an adjustable reservoir 74 containing hydraulic fluid. Fig. 10A illustrates when the band is in a non-constriction state, whereas Fig. 10B illustrates when the band is in a constriction state, in which the cavity 73 is expanded by hydraulic fluid supplied by the reservoir 74.

Figs. 31A, 31B, 31C and 31D are block diagrams of four differently operated hydraulic constriction devices. Fig. 31A shows the band 72 of Fig. 10A, the cavity 73 of which is in fluid communication with a reservoir 75. Fig. 31B shows the embodiment of Fig. 10A, in which the cavity 73 of the band 72 is in fluid communication with the reservoir 74 via an operation device in the form of a two-way pump 76. Fig. 11C shows an operation device in the form of a reverse servo device with a first closed device controlling a second device. The reverse servo device comprises an adjustable fluid supply reservoir 77 and an adjustable servo reservoir 78. The servo reservoir 78 controls a larger adjustable reservoir 79 which in connection with the band 72 applied around a portion of tubular tissue wall of a patient's organ varies the volume of the cavity 73 of the band 72, which in turn varies the constriction of the wall portion. Fig. 11D shows an embodiment identical to the embodiment of Fig. 11C, except that the larger reservoir 79 is omitted. Instead, the servo reservoir 78 is in fluid communication with the cavity of the band 72.

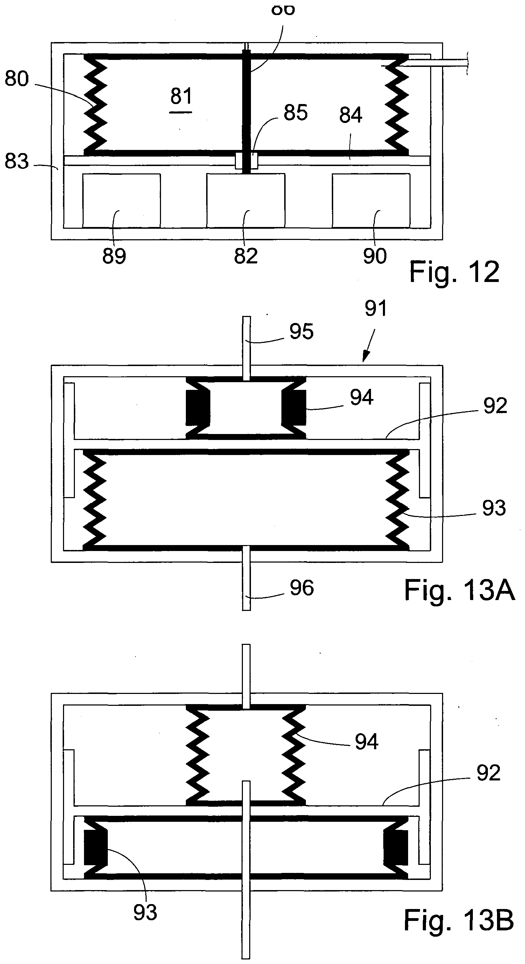

In all of the above embodiments according to Figs. 12A through 10B, stimulation devices may be provided to form constriction/stimulation units, in which the stimulation devices include a multiplicity of electrical elements 7 (indicated in Figs. 12A - 15, 18, 20 - 23, 26 - 11B) positioned on the constriction devices.

Fig. 12 is a cross-sectional view of a fluid supply device including a bellows reservoir 80 defining a chamber 81, the size of which is variable by an operation device comprising a remote controlled electric motor 82. The reservoir 80 and the motor 82 are placed in a housing 83. Moving a large wall 84 varies the chamber 81. The wall 84 is secured to a nut 85, which is threaded on a rotatable spindle 86. The spindle 86 is rotated by the motor 82. A battery 89 placed in the housing 83 powers the motor 82. A signal receiver 90 for controlling the motor 82 is also placed in the housing 83. Alternatively, the battery 89 and the signal receiver 90 may be mounted in a separate place. The motor 82 may also be powered with energy transferred from transmitted signals.

Where applicable, the fluid supply device of Fig. 12 may be used for supplying hydraulic fluid for the operation of the constriction devices described in this specification. For example, the fluid supply device of Fig. 12 may be substituted for the reservoir 74 in the embodiment according to Fig. 10A.

Figs. 13A and 13B show a reverse servo including a rectangular housing 91 and an intermediate wall 92, which is movable in the housing 91. A relatively large, substantially cylindrical bellows reservoir 93 is arranged in the housing 91 and is joined to the movable intermediate wall 92. Another cylindrical bellows reservoir 94, which is substantially smaller than reservoir 93, is arranged in the housing 91 at the other side of the intermediate wall 92 and is also joined to the wall 92. The small bellows reservoir 94 has a fluid supply pipe 95 and the large bellows reservoir 93 has a fluid supply pipe 96.

Referring to Fig. 13A, when a small amount of hydraulic fluid is conducted through the supply pipe 95 into the small bellows reservoir 94, the small bellows reservoir 94 expands and pushes the movable intermediate wall 92 towards the large bellows reservoir 93. As a result, the large bellows reservoir 93 is contracted by the intermediate wall 92, whereby a large amount of hydraulic fluid is forced out of the large bellows reservoir 93 through the supply pipe 96, as shown in Fig. 13B.

For example, the reverse servo of Figs. 13A and 13B may be used in the embodiment of Fig. 11C, wherein the small bellows reservoir 94 corresponds to the small servo reservoir 78 and the large bellows reservoir 93 corresponds to the large reservoir 79. Also, the reverse servo of Figs. 13A and 13B may be used in the embodiment of Fig. 10A and 10B, wherein the small bellows reservoir 94 is connected to the adjustable reservoir 74, and the large bellows reservoir 93 is connected to the cavity 73 of the band 72.

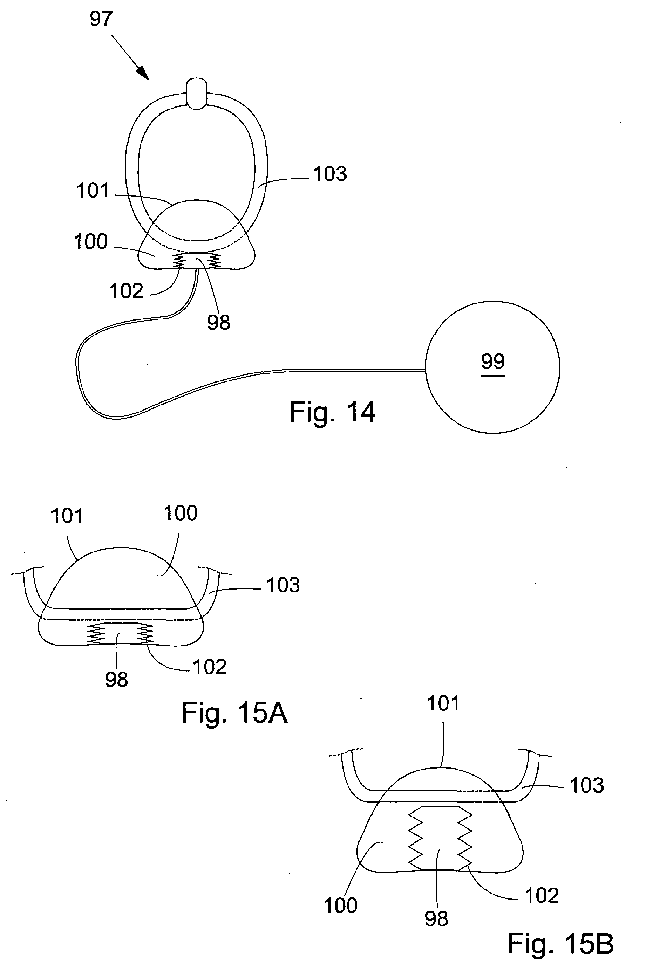

Fig. 14 schematically shows a hydraulically operable constriction device 97 of the device of the invention, which is similar to the embodiment shown in Fig. 10A, except that the hydraulic device is designed differently. Thus, the constriction device 97 includes a relatively small inflatable cavity 98, which is in fluid communication with a reservoir 99 containing hydraulic fluid, and a relatively large cavity 100, which is displaceable by small cavity 98. Small cavity 98 is adapted to displace large cavity 100 to constrict the patient's tubular wall portion when small cavity 98 is inflated and to displace large cavity 100 to release the wall portion when small cavity 98 is deflated. Thus, a relatively small addition of hydraulic fluid from reservoir 99 to small cavity 98 causes a relatively large increase in the constriction of the wall portion.

Large cavity 100 is defined by a contraction element in the form of a big balloon 101, which may be connected to an injection port (not shown) for calibration of the volume of large cavity 100. Adding fluid to or withdrawing fluid from the injection port with the aid of a syringe calibrates the volume of balloon 101. Small cavity 98 is defined by a small bellows 102 attached to an annular frame 103 of constriction device 97 and at the opposite end is attached to balloon 101.

Figs. 15A and 15B schematically illustrate the operation of constriction device 97, when annular frame 103 is applied around the tubular wall portion of the patient's organ. Referring to Fig. 15A, when small cavity 98 is deflated bellows 102 pulls balloon 101 inwardly into annular frame 103, so that constriction device 97 constricts the wall portion. Referring to Fig. 15B, when small cavity 98 is inflated bellows 102 pulls balloon 101 out of annular frame 103, so that constriction device 97 releases the wall portion.

As mentioned above, the constriction device and stimulation device can co-operate to actively move the egg in the oviduct of a patient's organ. This can be achieved using the constriction/stimulation unit shown in Fig. 2. Thus, in accordance with a first cooperation option, the clamping elements 5, 6 of the constriction device constricts the wall portion 8 without completely closing the oviduct, whereby the flow in the oviduct is restricted, and the control device 4 controls the electrical elements 7 to progressively stimulate the constricted wall portion in the downstream or upstream direction of the oviduct to cause progressive contraction of the wall portion 8 to move the egg in the oviduct.

In accordance with a second cooperation option, the constriction device constricts the wall portion so that the flow in the oviduct is restricted, and the control device 4 controls a few electrical elements 7 at one end of the elongate clamping elements 5, 6 to stimulate the constricted wall portion 8 to close the oviduct either at an upstream end or a downstream end of the wall portion 8. With the oviduct closed in this manner, the control device 4 controls the constriction device to increase the constriction of the wall portion, whereby the egg in the oviduct is moved downstream or upstream of the wall portion 8.

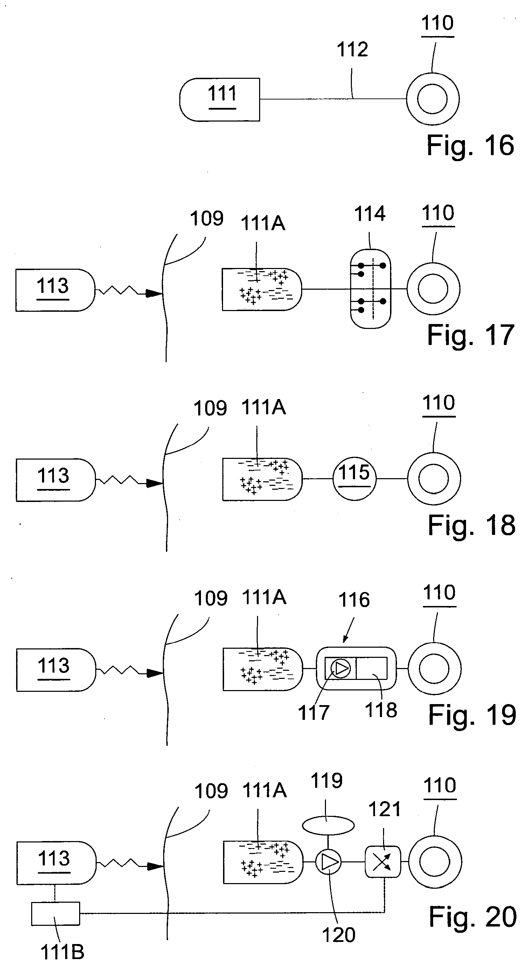

In another embodiment of the invention for performing the second cooperation option, the constriction device constricts the wall portion so that the flow in the oviduct is restricted, and the control device 4 controls the stimulation device to stimulate the constricted wall portion while the constriction device varies the constriction of the different areas of the wall portion, such that the wall portion is progressively constricted in the downstream or upstream direction of the oviduct. Figs. 16A - 16E show different operation stages of such an alternative embodiment, which comprises a constriction device 104 including two elongate constriction elements 105, 106 having convex surfaces 107, 108 that abut a length of the wall portion 8 on mutual sides thereof, and a multiplicity of electrical elements 7 (such as electrodes) that are positioned on the convex surfaces 107, 108. The control device 4 controls the electrical elements 7 during operation of the constriction device 104 and controls the elongate constriction elements 105, 106 to move relative to the tubular wall portion 8 so that the constriction elements 105, 106 progressively constrict the wall portion 8, as appears from Figs. 16A to 16D.

Thus, in an initial position of the constriction elements 105, 106 shown in Fig. 16A, the wall portion is not constricted by the constriction elements 105, 106 and the electrical elements 7 are not energized. Starting from this initial position, the control device 4 controls the constriction elements 105, 106 to swing the left ends of the constriction elements 105, 106 toward the wall portion (indicated by arrows) to constrict the tubular wall portion 8, see Fig. 16B, while energizing the electrical elements 7, so that the electrical elements 7 that contact the wall portion 8 contract the latter. Fig. 16C shows how the oviduct of the tubular wall portion 8 is completely closed by the thickened wall portion 8. Then, as shown in Fig. 16C, the control device 4 controls the constriction elements 105, 106 to move so that their right ends are moving towards each other (indicated by arrows), while the convex surfaces 107, 108 of the constriction elements 105, 106 are rolling on each other with the contracted wall portion 8 between them, see Fig. 16D. As a result, the bodily matter in the oviduct of the organ is forced to the right (indicated by a white arrow). When the constriction elements 105, 106 have rolled on each other to the position shown in Fig. 16E, the control device 4 controls the right ends of the constriction elements 105, 106 to move away from each other (indicated by arrows in Fig. 16E) to the initial position shown in Fig. 16A. The operation stages described according to Figs. 16A to 16E can be cyclically repeated a number of times until the desired amount of bodily matter has been moved in the oviduct of the organ in a peristaltic manner.

Alternatively, only one of the constriction elements 105, 106 can be provided with a convex surface, whereas the other constriction element has a plane surface that abuts the wall portion. It is also possible to use a single constriction element with a convex surface that presses the tubular portion 8 of the organ against a bone of the patient.

.Fig. 16 schematically shows a general embodiment of the device of the invention, in which energy is transferred to energy consuming components of the device implanted in the patient. The device of Fig. 16 comprises an implanted constriction/stimulation unit 110, which is operable to gently constrict a portion of a tubular tissue wall of a patient's organ and to stimulate different areas of the constricted portion to cause contraction of the wall portion. The constriction device of the constriction/stimulation unit 110 is capable of performing a reversible function, i.e., to constrict and release the wall portion, so that the constriction/stimulation unit 110 works as an artificial sphincter.

A source of energy 111 is adapted to supply energy consuming components of the constriction/stimulation unit 110 with energy via a power supply line 112. A wireless remote control or a subcutaneously implanted switch operable by the patient to switch on or off the supply of energy from the source of energy may be provided. The source of energy may be an implantable permanent or rechargeable battery, or be included in an external energy-transmission device, which may be operable directly by the patient or be controlled by a remote control operable by the patient to transmit wireless energy to the energy consuming components of the constriction/stimulation unit. Alternatively, the source of energy may comprise a combination of an implantable rechargeable battery, an external energy-transmission device and an implantable energy-transforming device for transforming wireless energy transmitted by the external energy-transmission device into electric energy for the charge of the implantable rechargeable battery.

Fig. 17 shows a special embodiment of the general embodiment of Fig. 16 having some parts implanted in a patient and other parts located outside the patient's body. Thus, in Fig. 17 all parts placed to the right of the patient's skin 109 are implanted and all parts placed to the left of the skin 109 are located outside the patient's body. An implanted energy-transforming device 111A of the device is adapted to supply energy consuming components of the constriction/stimulation unit 110 with energy via the power supply line 112. An external energy-transmission device 111 of the device includes a wireless remote control transmitting a wireless signal, which is received by a signal receiver incorporated in the implanted energy-transforming device 111A. The implanted energy-transforming device 111A transforms energy from the signal into electric energy, which is supplied via the power supply line 112 to the constriction/stimulation unit 110.

The device of Fig. 17 may also include an implanted rechargeable battery for energizing energy consuming implanted components of the device. In this case, the implanted energy-transforming device 111A also charges the battery with electric energy, as the energy-transforming device transforms energy from the signal into the electric energy.

A reversing device in the form of an electric switch 114, such as a microprocessor, is implanted in the patient for reversing the constriction device of the constriction/stimulation unit 110. The wireless remote control of the external energy-transmission device 113 transmits a wireless signal that carries energy and the implanted energy-transforming device 111A transforms the wireless energy into a current for operating the switch 114. When the polarity of the current is shifted by the energy-transforming-device 111A the switch 114 reverses the function performed by the constriction device of the constriction/stimulation unit 110.

Fig. 18 shows an embodiment of the invention including the energy-transforming device 11 1A, the constriction/stimulation unit 110 and an implanted operation device in the form of a motor 115 for operating the constriction device of the constriction/stimulation unit 110. The motor 115 is powered with energy from the energy-transforming device 111A, as the remote control of the external energy-transmission device 113 transmits a wireless signal to the receiver of the energy-transforming device 111A.

Fig. 19 shows an embodiment of the invention including the energy-transforming device 111A, the constriction/stimulation unit 110 and an implanted assembly 116 including a motor/pump unit 117 and a fluid reservoir 118. In this case the constriction device of the constriction/stimulation unit 110 is hydraulically operated, i.e., hydraulic fluid is pumped by the motor/pump unit 117 from the reservoir 118 to the constriction/stimulation unit 110 to constrict the wall portion, and hydraulic fluid is pumped by the motor/pump unit 117 back from the constriction/stimulation unit 110 to the reservoir 118 to release the wall portion. The implanted energy-transforming device 111A transforms wireless energy into a current, for powering the motor/pump unit 117.

Fig. 20 shows an embodiment of the invention comprising the external energy-transmission device 113.that controls the control unit 122 to reverse the motor 115 when needed, the constriction/stimulation unit 110, the constriction device of which is hydraulically operated, and the implanted energy-transforming device 111A, and further comprising an implanted hydraulic fluid reservoir 119, an implanted motor/pump unit 120, an implanted reversing device in the form of a hydraulic valve shifting device 121 and a separate external wireless remote control 111B. The motor of the motor/pump unit 120 is an electric motor. In response to a control signal from the wireless remote control of the external energy-transmission device 113, the implanted energy-transforming device 111A powers the motor/pump unit 120 with energy from the energy carried by the control signal, whereby the motor/pump unit 120 distributes hydraulic fluid between the reservoir 119 and the constriction device of the constriction/stimulation unit 110. The remote control 111B controls the shifting device 121 to shift the hydraulic fluid flow direction between one direction in which the fluid is pumped by the motor/pump unit 120 from the reservoir 119 to the constriction device of the constriction/stimulation unit 110 to constrict the wall portion, and another opposite direction in which the fluid is pumped by the motor/pump unit 120 back from the constriction device of the constriction/stimulation unit 110 to the reservoir 119 to release the wall portion.

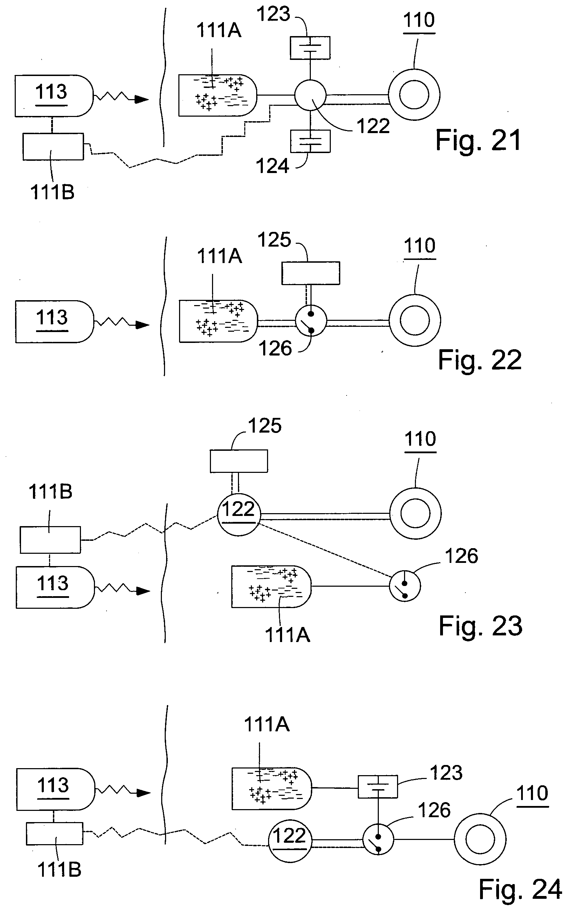

Fig. 21 shows an embodiment of the invention including the energy-transforming device 111A and the constriction/stimulation unit 110. A control unit 122, an accumulator 123 and a capacitor 124 are also implanted in the patient. A separate external wireless remote control 111B controls the control unit 122. The control unit 122 controls the energy-transforming device 111A to store electric energy in the accumulator 123, which supplies energy to the constriction/stimulation unit 110. In response to a control signal from the wireless remote control 111B, the control unit 122 either releases electric energy from the accumulator 123 and transfers the released energy via power lines, or directly transfers electric energy from the energy-transforming device 111A via the capacitor 124, which stabilises the electric current, for the operation of the constriction/stimulation unit 110.

In accordance with one alternative, the capacitor 124 in the embodiment of Fig. 21 may be omitted. In accordance with another alternative, the accumulator 123 in this embodiment may be omitted.

Fig. 22 shows an embodiment of the invention including the energy-transforming device 111A, the constriction/stimulation unit 110. A battery 125 for supplying energy for the operation of the constriction/stimulation unit 110 and an electric switch 126 for switching the operation of the constriction/stimulation unit 110 are also implanted in the patient. The switch 126 is operated by the energy supplied by the energy-transforming device 111A to switch from an off mode, in which the battery 125 is not in use, to an on mode, in which the battery 125 supplies energy for the operation of the constriction/stimulation unit 110.

Fig. 23 shows an embodiment of the invention identical to that of Fig. 43, except that a control unit 122 also is implanted in the patient. A separate external wireless remote control 111B controls the control unit 122. In this case, the switch 126 is operated by the energy supplied by the energy-transforming device 111A to switch from an off mode, in which the wireless remote control 111B is prevented from controlling the control unit 122 and the battery 125 is not in use, to a standby mode, in which the remote control 111B is permitted to control the control unit 122 to release electric energy from the battery 125 for the operation of the constriction/stimulation unit 110.

Fig. 24 shows an embodiment of the invention identical to that of Fig. 44, except that the accumulator 123 is substituted for the battery 125 and the implanted components are interconnected differently. In this case, the accumulator 123 stores energy from the energy-transforming device 111A. In response to a control signal from the wireless remote control 111B, the implanted control unit 122 controls the switch 126 to switch from an off mode, in which the accumulator 123 is not in use, to an on mode, in which the accumulator 123 supplies energy for the operation of the constriction/stimulation unit 110.

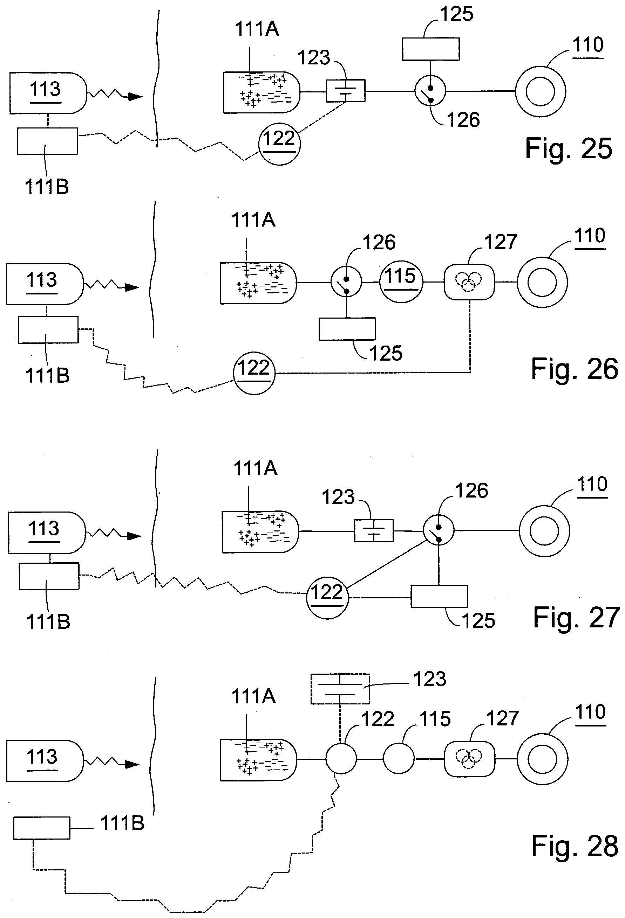

Fig. 25 shows an embodiment of the invention identical to that of Fig. 45, except that the battery 125 also is implanted in the patient, and the implanted components are interconnected differently. In response to a control signal from the wireless remote control 111B, the implanted control unit 122 controls the accumulator 123, which may be a capacitor, to deliver energy for operating the switch 126 to switch from an off mode, in which the battery 125 is not in use, to an on mode, in which the battery 125 supplies electric energy for the operation of the constriction/stimulation unit 110.

Alternatively, the switch 126 may be operated by energy supplied by the accumulator 123 to switch from an off mode, in which the wireless remote control 111B is prevented from controlling the battery 125 to supply electric energy and the battery 125 is not in use, to a standby mode, in which the wireless remote control 111B is permitted to control the battery 125 to supply electric energy for the operation of the constriction/stimulation unit 110.

Fig. 26 shows an embodiment of the invention identical to that of Fig. 43, except that a motor 115, a mechanical reversing device in the form of a gearbox 127 and a control unit 122 for controlling the gearbox 127 also are implanted in the patient. A separate external wireless remote control 111B controls the implanted control unit 122 to control the gearbox 127 to reverse the function performed by the constriction device (mechanically operated) of the constriction/stimulation unit 110.

Fig. 27 shows an embodiment of the invention identical to that of Fig. 46, except that the implanted components are interconnected differently. Thus, in this case, the battery 125 powers the control unit 122 when the accumulator 123, suitably a capacitor, activates the switch 126 to switch to an on mode. When the switch 126 is in its on mode the control unit 122 is permitted to control the battery 125 to supply, or not supply, energy for the operation of the constriction/stimulation unit 110.

Fig. 28 shows an embodiment of the invention identical to that of Fig. 39, except that a gearbox 127 that connects the motor 115 to the constriction/stimulation unit 110, and a control unit 122 that controls the energy-transforming device 111A to power the motor 115 also are implanted in the patient. There is a separate external wireless remote control 111B that controls the control unit 122 to reverse the motor 115 when needed.

Optionally, the accumulator 123 shown in Fig. 21 may be provided in the embodiment of Fig. 49, wherein the implanted control unit 122 controls the energy-transforming device 111A to store the transformed energy in the accumulator 123. In response to a control signal from the wireless remote control 111B, the control unit 122 controls the accumulator 123 to supply energy for the operation of the constriction/stimulation unit 110.

Those skilled in the art will realize that the above various embodiments according to Figs. 17-28 could be combined in many different ways. For example, the energy operated switch 114 could be incorporated in any of the embodiments of Figs. 18, 21-28, the hydraulic shifting device 121 could be incorporated in the embodiment of Fig. 21, and the gearbox 127 could be incorporated in the embodiment of Fig. 39. The switch 114 may be of a type that includes electronic components, for example a microprocessor, or a FGPA (Field Programmable Gate Array) designed for switching. Alternatively, however, the energy operated switch 114 may be replaced by a subcutaneously implanted push button that is manually switched by the patient between "on" and "off".

Alternatively, a permanent or rechargeable battery may be substituted for the energy-transforming devices 111A of the embodiments shown in Figs. 38-49.

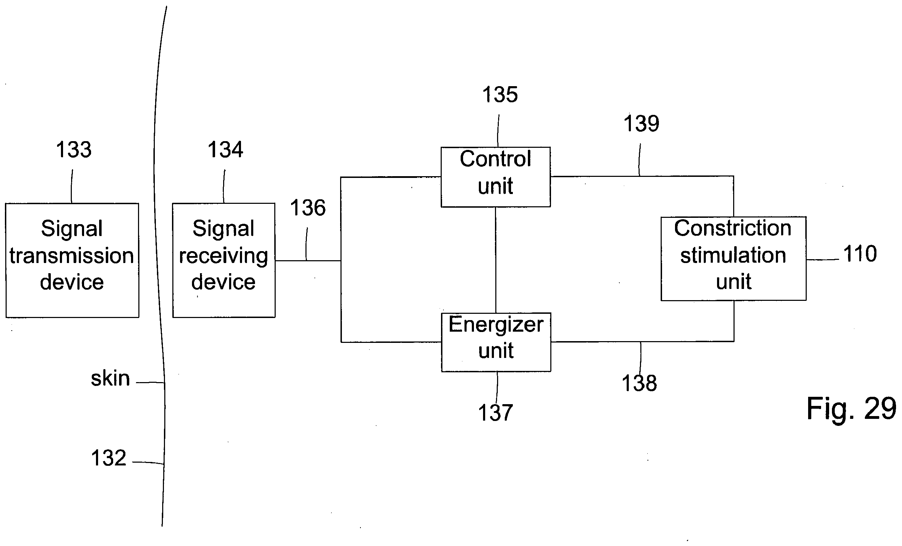

Fig. 29 shows basic parts of a remote control of the device of the invention for controlling the constriction/stimulation unit 110. In this case, the stimulation device of the constriction/stimulation unit stimulates the wall portion with electric pulses. The remote control is based on wireless transmission of electromagnetic wave signals, often of high frequencies in the order of 100 kHz - 1 GHz, through the skin 132 of the patient. In Fig. 29, all parts placed to the left of the skin 132 are located outside the patient's body and all parts placed to the right of the skin 132 are implanted.

An external signal-transmission device 133 is to be positioned close to a signal-receiving device 134 implanted close to the skin 132. As an alternative, the signal-receiving device 134 may be placed for example inside the abdomen of the patient. The signal-receiving device 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The signal transmission device 133 comprises a coil having about the same size as the coil of the signal-receiving device 134 but wound with a thick wire that can handle the larger currents that is necessary. The coil of the signal transmission device 133 is tuned to the same specific high frequency as the coil of the signal-receiving device 134.

The signal-transmission device 133 is adapted to send digital information via the power amplifier and signal-receiving device 134 to an implanted control unit 135. To avoid that accidental random high frequency fields trigger control commands, digital signal codes are used. A conventional keypad placed on the signal transmission device 133 is used to order the signal transmission device 133 to send digital signals for the control of the constriction/stimulation unit. The signal transmission device 133 starts a command by generating a high frequency signal. After a short time, when the signal has energized the implanted parts of the control device, commands are sent to operate the constriction device of the constriction/stimulation unit 110 in predefined steps. The commands are sent as digital packets in the form illustrated below.
Start pattern, 8 bits
Command, 8 bits
Count, 8 bits
Checksum, 8 bits

The commands are sent continuously during a rather long time period (e.g., about 30 seconds or more). When a new constriction or release step is desired, the Count byte is increased by one to allow the implanted control unit 135 to decode and understand that another step is demanded by the signal transmission device 133. If any part of the digital packet is erroneous, its content is simply ignored.

Through a line 136, an implanted energizer unit 137 draws energy from the high frequency electromagnetic wave signals received by the signal-receiving device 134. The energizer unit 137 stores the energy in a source of energy, such as a large capacitor, powers the control unit 135 and powers the constriction/stimulation unit 110 via a line 138.

The control unit 135 comprises a demodulator and a microprocessor. The demodulator demodulates digital signals sent from the signal transmission device 133. The microprocessor receives the digital packet, decodes it and sends a control signal via a signal line 139 to control the constriction device of the constriction/stimulation unit 110 to either constrict or release the wall portion of the patient's organ depending on the received command code.

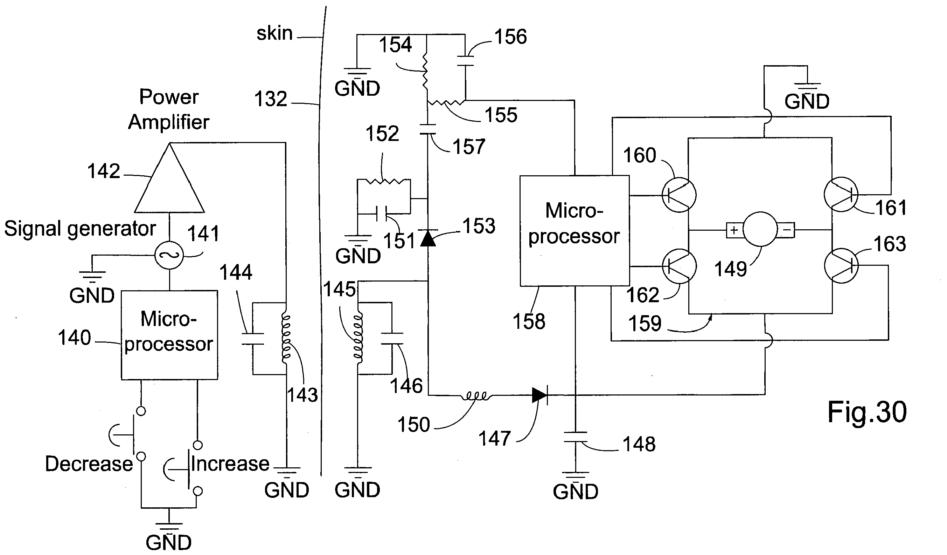

Fig. 30 shows a circuitry of an embodiment of the invention, in which wireless energy is transformed into a current. External components of the circuitry include a microprocessor 140, a signal generator 141 and a power amplifier 142 connected thereto. The microprocessor 140 is adapted to switch the signal generator 141 on/off and to modulate signals generated by the signal generator 141 with digital commands. The power amplifier 142 amplifies the signals and sends them to an external signal-transmitting antenna coil 143. The antenna coil 143 is connected in parallel with a capacitor 144 to form a resonant circuit tuned to the frequency generated by the signal generator 141.

Implanted components of the circuitry include a signal receiving antenna coil 145 and a capacitor 146 forming together a resonant circuit that is tuned to the same frequency as the transmitting antenna coil 143. The signal receiving antenna coil 145 induces a current from the received high frequency electromagnetic waves and a rectifying diode 147 rectifies the induced current, which charges a storage capacitor 148. The storage capacitor 148 powers a motor 149 for driving the constriction device of the constriction/stimulation unit 110. A coil 150 connected between the antenna coil 145 and the diode 147 prevents the capacitor 148 and the diode 147 from loading the circuit of the signal-receiving antenna 145 at higher frequencies. Thus, the coil 150 makes it possible to charge the capacitor 148 and to transmit digital information using amplitude modulation.

A capacitor 151 and a resistor 152 connected in parallel and a diode 153 form a detector used to detect amplitude modulated digital information. A filter circuit is formed by a resistor 154 connected in series with a resistor 155 connected in series with a capacitor 156 connected in series with the resistor 154 via ground, and a capacitor 157, one terminal of which is connected between the resistors 154,155 and the other terminal of which is connected between the diode 153 and the circuit formed by the capacitor 151 and resistor 152. The filter circuit is used to filter out undesired low and high frequencies. The detected and filtered signals are fed to an implanted microprocessor 158 that decodes the digital information and controls the motor 149 via an H-bridge 159 comprising transistors 160, 161, 162 and 163. The motor 149 can be driven in two opposite directions by the H-bridge 159.

The microprocessor 158 also monitors the amount of stored energy in the storage capacitor 148. Before sending signals to activate the motor 149, the microprocessor 158 checks whether the energy stored in the storage capacitor 148 is enough. If the stored energy is not enough to perform the requested operation, the microprocessor 158 waits for the received signals to charge the storage capacitor 148 before activating the motor 149.

Alternatively, the energy stored in the storage capacitor 148 may only be used for powering a switch, and the energy for powering the motor 149 may be obtained from another implanted energy source of relatively high capacity, for example a battery. In this case the switch is adapted to connect the battery to the motor 149 in an on mode when the switch is powered by the storage capacitor 148 and to keep the battery disconnected from the motor 149 in a standby mode when the switch is not powered.

While the invention has been described in connection with what is presently considered to be the most practical and preferred embodiment, it is to be understood that the invention is not to be limited to the disclosed embodiment, but on the contrary, is intended to cover various modifications and equivalent arrangements included within the spirit and scope of the appended claims.

### ITEMIZED LIST OF EMBODIMENTS

1. A system for treating a female mammal or human patient to promote pregnancy comprising;
   a restriction device adapted to postoperatively be adjusted to restrict and release an oviduct of the patient.
2. The system according to item 1, wherein the restriction device is adapted to provide a restriction of the oviduct to accumulate at least one egg released from the ovary in the oviduct.
3. The system according to item 1, wherein the restriction device is adapted to provide a release of the oviduct when pregnancy is wanted.
4. The system according to item 1, wherein the restriction device is adapted to be adjusted from outside the patients body to restrict and release the oviduct passageway.
5. The system according to item 4, wherein the restriction device is adapted to be adjusted from outside the patients body non-invasively.
6. The system according to item 1, wherein the restriction device is adapted to be adjusted by manual manipulation.
7. The system according to item 1, wherein the restriction device is adapted to be adjusted by electrical or magnetic power.
8. The system according to item 1, wherein the restriction device is adapted to be adjusted by hydraulic power.
9. The system according to item 8, wherein the hydraulic power comprising at least one subcutaneously placed reservoir controlled by the patient.
10. The system according to item 1, wherein the restriction device is adapted to be adjusted reversible.
11. The system according to item 1, wherein the restriction device is adapted to provide a restriction to accumulate at least one egg released from the ovary in the oviduct for a predetermined period of time, and
   releasing the restriction when convenient for the patient to get pregnant.
12. The system according to item 11, wherein the predetermined period of time is adapted to increase the likelihood of pregnancy.
13. The method according to item 11, wherein the predetermined period of time is between 0 and 2 days.
14. The method according to item 11, wherein the predetermined period of time is between 0 and 2 days.

### MORE THAN ONE RESTRICTION AREA

15. The system according to any of item 1-14, wherein said restriction device comprises a more than one restriction area, wherein said restriction device being adapted to change the restriction area over time.
16. The system according to item 15, wherein the change of the restriction area is adapted to prevent any damage to the oviduct still keeping the oviduct closed accumulating any egg to at a defined time be released to pas down to the uterus, thus increasing the likelihood of pregnancy.
17. The system according to item 15, comprising a hydraulic restriction device with two or more restriction areas.
18. The system according to item 17, comprising a hydraulic reservoir connected to said restriction device, wherein the hydraulic restriction device with two or more restriction areas is connected to the reservoir with hydraulic fluid adapted to move fluid to or from the restriction device and further comprising at least one valve to direct the fluid individually to or from the restriction areas to restrict or release the restriction areas individually.
19. The system according to item 17, comprising two or more hydraulic reservoirs connected one each to the different areas of said restriction device, wherein the hydraulic restriction device with two or more restriction areas is connected to the two or more reservoirs with hydraulic fluid, said reservoirs is adapted to move fluid to or from said restriction areas individually to or from each of the connected restriction areas to restrict or release the restriction in the individual restriction area.
20. The system according to item 17, wherein each of the hydraulic restriction areas is adapted to be restricted for a predetermined time period with some overlap in time and adapted to first restrict the restriction area closest to the ovary then changing restriction area to the next one towards the uterus.
21. The system according to item 17, wherein each of the hydraulic restriction areas is adapted to be regulated by manual manipulation of said reservoirs.
22. The system according to item 15, comprising a stimulation device with two or more restriction areas in combination with a hydraulic or mechanical restriction device.
23. The system according to item 22, wherein the hydraulic or mechanical restriction device is only partly restricting the oviduct and the stimulation device stimulates to completely restrict the oviduct.
24. The system according to item 23, wherein the stimulation device stimulates different areas of the oviduct to vary the area of the oviduct that is completely restricted.
25. The system according to of item 15, wherein the change of the restriction area is adapted to cause a peristaltic wave like restriction wave in the direction towards the ovary to prevent the egg being transported down to the uterus.
26. The system according to item 1, wherein the restriction device is adapted to effect a transport of the at least one egg to the uterus upon release of the oviduct.
27. The system according to item 26, wherein the restriction device is adapted to cause a peristaltic wave like restriction wave in the direction towards the uterus.
28. A system of promoting pregnancy according to item 15, adapted to restrict a first part or area of an oviduct of the patient to provide a restriction to accumulate at least one egg released from the ovary in the oviduct for a predetermined period of time, and adapted to restrict a second part of the oviduct and thereafter release the restriction of the first part.
29. A system according to item 28, further adapted to release the restriction of the second part, thereby allow transport of the egg down to the uterus.
30. A system according to item 28, adapted to restrict a third part of the oviduct and thereafter release the restriction of the second part.
31. A system according to item 30, further adapted to release the restriction of the third part thereby allow transport of the egg down to the uterus.
32. A system according to item 30, adapted to restrict a fourth part of the oviduct and thereafter release the restriction of the third part.
33. A system according to item 32, further adapted to release the restriction of the fourth part, thereby allow transport of the egg down to the uterus.
34. A system according to item 28, adapted to restrict the first part of the oviduct closest to the ovary allow the second restriction being restricted without interfering with any accumulated egg.
35. A system according to item 29-33, adapted to restrict a part of the oviduct closest to the ovary allow the next restriction being restricted without interfering with any accumulated egg.
36. A system according to item 1-35, adapted to have
   more than two restricting areas and varying the restricting area while
   at least one restricting area is closed when the device being in restriction mode.
37. A system according to item 36, adapted to restrict the restriction areas in consecutive order starting with the restriction area, part of the oviduct, closest to the ovary and thereafter restrict any new area one step closer to the uterus and further adapted to overlap in time the restriction of two consecutive restriction areas thereby adapted to restrict without interfering with any accumulated egg.

### PERISTALTIC LIKE WAVE OVIDUCT WALL MOVEMENTS

38. The device according to item 1, wherein the restriction device is adapted to only partly restricting the oviduct and to create peristaltic wave like movements of a part of the oviduct(s) wall to prevent the transport of an egg in an oviduct to the uterus of the human or mammal patient, and therefore accumule at least one egg released from the ovary in the oviduct for a predetermined period of time, and wherein the spermie is able to reach the egg during the time the egg is accumulated because the oviduct is only partly restricted, and the restriction device further adapted to release the egg, controlled from outside the body, to allow the at least on egg in the oviduct a transport to the uterus.
39. The device according to item 1, wherein said device is adapted to comprise at least one restricted area adapted to at least partly restrict and change the restriction area over time.
40. The device according to item 39, wherein said device is adapted to comprise two or more restricted areas adapted to at least partly restrict different portions of the oviduct and change the restriction area over time.
41. The device according to item 40, wherein the change of the restriction area is adapted to cause a peristaltic wave like wave in the direction towards the ovary to prevent the egg being transported down to the uterus.
42. The device according to item 38-41, comprising a hydraulic device adapted to cause said peristaltic wave like restriction wave.
43. The device according to item 38-41, comprising a mechanical device adapted to cause said peristaltic wave like restriction wave.
44. The device according to item 38-41, comprising a stimulation device adapted to cause said peristaltic wave like restriction wave.
45. The device according to item 38-44, wherein the device is adapted to allow a transport of the at least one egg to the uterus upon release of the oviduct.
46. The device according to item 45, wherein the device is adapted to cause a another peristaltic wave like restriction wave in the opposite direction towards the uterus.
47. The device according to item 39-40, wherein the device is adapted to be adjusted by manual manipulation.
48. The device according to item 39-40, wherein the device is adapted to be adjusted by electrical or magnetic power.
49. The device according to item 39-40, wherein the restriction areas is only partly restricted to allow the spermie to reach the egg and the device adapted to prevent the egg to temporary reach the uterus with the peristaltic wave, to accumulate the egg temporary in the oviduct to allow increased likelihood for pregnancy to prolong the time period the egg being reachable by the spermie.
50. The device according to item 39-40, wherein the restriction areas is completely restricted to prevent the egg to temporary reach the uterus, to accumulate the egg temporary in the oviduct to allow increased likelihood for pregnancy when released.
51. The device according to item 1-50, wherein the restriction device is adapted to and intended to repeatedly every month repeat the post-operational and non-invasive regulation of the device actively reversible to prevent transport of an egg from the ovary to the uterus in the oviduct of the patient and reverse the function by allowing the normal transport of the egg from the ovary to the uterus.
52. The device according to any of items 38 or 40-51, wherein the device comprises a mechanical device adapted to cause said peristaltic like wave towards the ovary.
53. The device according to any of items 38 or 40-51, wherein the device comprises a hydraulic device adapted to cause said peristaltic like wave towards the ovary.
54. The device according to any of items 38 or 40-51, wherein said device comprises a stimulation device adapted to cause said peristaltic like wave towards the ovary.

### MOVEMENT DEVICE

55. The device according to any of item 15-54, comprising a movement device adapted to move the egg out of a varied upcoming new restricted area before a new area is restricted.
56. The device according to item 55, wherein the movement device is the same device as the restriction device, adapted to work differently when restricting or causing movements of the egg.
57. The device according to item 55, wherein the movement device is a different device as the restriction device adapted to work differently when restricting or causing movements of the egg.
58. The device according to any of item 55-57, wherein the movement device cases vibration or wave like movements in the oviduct wall thereby causing movements of the egg.
59. The system according to any of item 55-58, wherein the movement device comprises a mechanical movement device.
60. The system according to any of item 55-58, wherein the movement device comprises a hydraulic movement device.
61. The system according to any of item 55-58, wherein said movement device comprises a stimulation device.
62. The system according to any of item 55-58, wherein said movement device comprises a stimulation device combined with a mechanical or hydraulic movement device.
63. The system according to any of item 59-61, wherein the movement device comprises any combination of movement devices.

### DIFERENT RESTRICTION EMBODIMENTS

64. The system according to any of item -63, wherein the restriction device comprises a mechanical restriction device.
65. The system according to any of item -63, wherein the restriction device comprises a hydraulic restriction device.
66. The system according to any of item -63, wherein said restriction device comprises a stimulation device.
67. The system according to any of item -63, wherein said restriction device comprises a stimulation device combined with a mechanical or hydraulic restriction device.
68. The system according to any of item 64-66, wherein the restriction device comprises any combination of restriction devices.

### CONTROL AND SENSOR INPUT

69. The system according to any of item -68, comprising a control device to control the restriction device.
70. The system according to item 69, wherein the control device is adapted to control the restriction device from outside the body of the patient.
71. The system according to item 69, wherein the control device are adapted to be operated from outside the human body.
72. The system according to item 69, wherein the control device comprises an internal control unit adapted to be implanted in the body of the patient, wherein the control of the restriction device is made by said internal control unit.
73. The system according to item 69 or 72, comprising a sensor sensing at least one physical parameter of the patient.
74. The system according to item 73, wherein the internal control unit controls the restriction device to restrict said oviduct based on information from said sensor.
75. The system according to item 70, comprising a sensor sensing at least one physical parameter of the patient, wherein the control device controls the restriction device based on information from said sensor.
76. The system according to item 69 or 72, comprising a sensor sensing at least one functional parameter of the device.
77. The system according to item 76, wherein the internal control unit controls the restriction device to restrict said oviduct based on information from said sensor.
78. The system according to item 70, comprising a sensor sensing at least one functional parameter of the device, wherein the control device controls the restriction device based on information from said sensor.
79. The system according to item73 or 75. wherein said sensor sensing a hormone level or a temperature.
80. The system according to item 76 or 78, wherein said sensor sensing a electrical parameter or a pressure related to the device.
81. The system according to any of item 1-80, comprising an energy source powering the restriction device, wherein said energy source being an internal energy source adapted to being chargeable by wireless energy, and further comprising a transmitter for transmitting wireless energy to charge said internal energy source.
82. The system according to any of item 1-80, adapted to send feedback information from inside the body to the outside thereof to give feed back related to any functional parameter of the device or physical parameter of the patient.
83. The system according to item 81, adapted to send feedback information from inside the body to the outside thereof to give feed back related to any functional parameter of the device or physical parameter of the patient.
84. The system according to item 83, wherein the functional parameter of the device is correlated to the transfer of energy for charging the internal energy source.
85.The system according to item 84, wherein the functional parameter of the device is the energy balance, the balance between the energy received and the energy used including accumulated by the device.
86.The system according to item 85, wherein the energy balance is the balance between an energy reception rate and an energy using including accumulating rate.
87. The system according to any of item 69-80, wherein the control device comprises a mechanical control device.
88. The system according to any of item 69-80, wherein the control device comprises a hydraulic control device.
89. The system according to any of item 69-80, wherein said control device comprises a stimulation device.

### POWER SUPPLY AND REMOTE CONTROL

90. The system according to any of item -89, comprising an energy source powering the restriction device.
91. The system according to any of item 69-80 and 87-89, wherein the control device comprises a wireless energy transmitter for wireless transfer of energy to power the restriction device during energy transfer.
92. The system according to item 90, wherein the system comprises a internal energy source to power the restriction device.
93. The system according to item 92, comprising a wireless energy transmitter for wireless transfer of energy to charge the internal energy source, being rechargeable.
94. The system according to item 92, wherein the internal energy source is controlled from outside the patients body.
95. The system according to item 94, wherein the internal energy source is controlled with a wireless remote control.
96. The system according to item 94, wherein the internal energy source is controlled with a switch implanted in the human body adapted to be regulated from outside the body.

### METHOD

97. A method of avoiding pregnancy of a female mammal or human patient, comprising the following steps:
   restricting an oviduct of the patient postoperatively to provide a restriction to accumulate at least one egg released from the ovary in the oviduct for a predetermined period of time, and
   releasing the restriction to admit any egg in the oviduct a transport to the uterus controlling the restricting and releasing procedures from outside the patients body.
98. The method according to item 70, wherein the predetermined period of time is adapted to promote pregnancy.
99 The method according to item 70, wherein the predetermined period of time is between 0 and 2 days.
100. A method for placing and controlling two implanted restriction devices avoiding pregnancy in a human or mammal patient, the method comprising the steps of:
   - inserting a needle like tube into the abdomen of the patients body,
   - using the tube like needle like to fill the abdomen with gas thereby expanding the abdominal cavity,
   - placing at least two laparoscopic trocars in the patient's body,
   - inserting a camera through one of the trocars into the abdomen,
   - inserting at least one dissecting tool through a trocar and dissecting an area of at least one portion of the two oviducts of the patient,
   - placing two implanted restriction devices, on each of the two oviducts
   - adjusting the restriction devices after the operation at a time convenient to not get pregnat thus,
   - controlling the adjustment from outside the patients body and post-operatively
   - restricting at least partly the two oviducts and thereby
   - accumulating at lest one egg in the oviduct and
   - releasing the at least one egg to allow transport down to the uterus at a time when pregnacy want to be achieved
101. A method according to item 100, wherein the step of restricting the oviduct at least partly thereby accumulating at least one egg may include the steps of: - restricting the oviduct partly and
   - causing an peristaltic wave like movement of the oviduct wall in the direction towards the ovary still only partly restricted to prevent transport of any egg down to the uterus thus accumulating said egg in the oviduct thereby
   - allowing a spermie to be able to reach the egg during the accumulation period.

### Method different restriction areas

102. A method of preventing pregnancy according to item 97 or 100 , wherein restricting the oviduct comprising the following steps:
   - restricting a first part or area of an oviduct of the patient to provide a restriction to accumulate at least one egg released from the ovary in the oviduct for a predetermined period of time,
   - restricting a second part of the oviduct, and
   - releasing the restriction of the first part.
103. A method according to item 102, comprising the following steps:
   - releasing the restriction of the second part, and
   - allowing transport of the egg down to the uterus.
104. A method according to item 102, comprising the following steps:
   - restricting a third part of the oviduct, and
   - releasing the restriction of the second part.
105. A method according to item 104, comprising the following steps:
   - releasing the restriction of the third part, and
   - allowing transport of the egg down to the uterus.
106. A method according to item 104, comprising the following steps:
   - restricting a fourth part of the oviduct, and
   - releasing the restriction of the third part.
107. A method according to item 106, comprising the following steps:
   - releasing the restriction of the fourth part, and
   - allowing transport of the egg down to the uterus.
108. A method according to item 102, comprising the following steps:
   wherein the restricted first part of the oviduct being closer to the ovary
   - allowing the second restriction being restricted without interfering with any accumulated egg.
109. A method according to item 97 or 100, comprising the following steps:
   - having more than two restricting areas, and
   - varying the restricting area while
   - keeping at least one restricting area closed when restriction wants to be achieved
110. A method according to item 109, comprising the following steps:
   the restriction areas being restricted in consecutive order starting with the restriction area, closest to the ovary thereafter,
   - restricting any new area one step closer to the uterus,
   - overlapping in time the restriction of two consecutive restriction areas thereby, and
   - restricting without interfering with any accumulated egg
111. A method of promoting pregnancy of a female human or mammal patient, comprising the following steps:
   - restricting a first part of an oviduct of the patient to provide a restriction to accumulate at least one egg released from the ovary in the oviduct for a predetermined period of time, and
   - moving the accumulated egg by a movement device in the oviduct towards the ovary, away from the second restriction area, on the ovary side of the first restriction,
   - allowing a second part of the oviduct closer to the ovary being restricted without interfering with any accumulated egg,
   - releasing the restriction of the first part,
   - repeating the restriction of the first part,
   - releasing the restriction of the second part, and
   - allowing the oviduct to recover between restriction intervals.
112.The method according to item 111,
   - repeating moving any accumulated egg by said movement device away in the oviduct towards the ovary from the not yet restricted second area, repeating
   - allowing a second part of the oviduct closer to the ovary being restricted without interfering with any accumulated egg, further repeating,
   - releasing the restriction of the first part, and
   - repeating the complete procedure allowing the oviduct to recover between restriction intervals.
113.The method according to item 111, wherein more than two restricted areas are varied to keep the oviduct closed allowing the restricted area to recover between restriction intervals and wherein the restriction areas is adapted to be varied between three or more areas always keeping the oviduct closed.
114.The method according to item 113, comprising the step of:
   - moving any accumulated egg by said movement device away in the oviduct towards the ovary from any new upcoming restriction area at a time to avoid interfering with any accumulated egg within the restriction.
115. The method according to item 100, comprising the step of:
   releasing the restriction after a determined time period to admit the at least on egg in the oviduct to allow a transport of the at least one egg to the uterus at a time convenient to not get pregnant.
116.The method according to item 97 or 100, wherein the device is adapted to have the restriction areas varied over time to avoid any damage to the oviduct still keeping the oviduct completely restricted.
117.The method according to item 116, comprising a movement device, wherein the movement device is adapted to move the egg out of a varied upcoming new restricted region before a new area is restricted.
118.The method e according to item 117, wherein the movement device is the same device as the restriction device, adapted to work differently when restricting or causing movements of the egg.
119.The method according to item 117, wherein the movement device is a different device as the restriction device adapted to work differently when restricting or causing movements of the egg.
120. The method according to any of item 117-119, wherein the movement device cases vibration or wave like movements in the oviduct wall.
121. The method according to item 115, wherein the predetermined period of time is adapted to prevent pregnancy.
122.The method according to item 115, wherein the predetermined period of time is between 2 and 30 days.
123. The method according to item 115, wherein the predetermined period of time is more than 30 days.
124. The method according to any of item 117-119, wherein said movement device comprises a vibrating device, for causing a vibration of at least a part of the wall of said oviduct causing movement of any accumulated egg, said movement being repeated.
125. The method according to any of item 117-119, wherein said movement device comprises a mechanical device.
126. The method according to any of item 117-119, wherein said movement device comprises hydraulic device.
127. The method according to any of item 117-119, wherein said movement device comprises stimulation device.
128. The method according to item 125-127, wherein said movement device comprises a combined device.
129. A method of promoting pregnancy of a female patient, comprising the following steps:
   preventing the transport of an egg in an oviduct to the uterus of the human or mammal patient, and
   accumulating at least one egg released from the ovary in the oviduct for a predetermined period of time, by
      - casing a peristaltic like wave movement of a part of the oviduct(s) wall preventing the egg being transported down to the uterus still only partly restricting the oviduct,
      - allowing the spermie to reach the egg during the time the egg is accumulated, and
      - releasing the egg non-operatively to admit the at least on egg in the oviduct to allow a transport of the at least one egg to the uterus.
130. The method according to item 129, wherein the predetermined period of time is adapted to promote pregnancy.
131. The method according to item 130, wherein the predetermined period of time is between 0 and 2 days.
132. The method according to item 130, wherein the predetermined period of time is between 0 and 2 days.
133. A method for placing and controlling an implanted device achieving an increased likelihood of pregnancy in a human or mammal patient, the method comprising the steps of:
   - inserting a tube like needle into the abdomen of the patients body,
   - using the tube like needle like to fill the abdomen with gas thereby expanding the abdominal cavity,
   - placing at least two laparoscopic trocars in the patient's body,
   - inserting a camera through one of the trocars into the abdomen,
   - inserting at least one dissecting tool through a trocar and dissecting an area of at least one portion of the two oviducts of the patient,
   - placing two parts of the implanted device, one each on the two oviducts
   - finishing the operation and withdrawing the instruments after eventual suturing and thereafter postoperatively:
   - adjusting the device after the operation at a time relevant to get pregnat,
   - controlling the adjustment from outside the patients body and thereby
   - preventing flow of any egg to reach the uterus in the two oviducts during a few days in relation to the ovulation period and thereby increasing the likelihood of the right timing for to get pregnant and thereby
   - accumulating any egg released from the ovary in the oviduct(s) by
   - casing a peristaltic like wave movement of a part of the oviduct(s) wall preventing the egg being transported down to the uterus still only partly restricting the oviduct,
   - allowing the spermie to reach the egg during the time the egg is accumulated
   - releasing any egg in the oviduct noninvasively to allow the egg to in a normal way be transported down to the uterus.
134. The method according to item 129 or 133, wherein said device comprises a mechanical device.
135. The method according to item 129 or 133, wherein said restriction device comprises hydraulic device.
136. The method according to item 135 wherein said hydraulic restriction device comprises a reservoir, for moving gas or fluid to or from said device.
137. The method according to item 136, wherein said reservoir is placed subcutaneously for
   - being reached by the patients hand for
   - moving fluid manually to or from said device.
138. The method according to item 129 or 133, wherein said device - being powered by an internal energy source for preventing the transport of an egg to the uterus, comprising a control device controlling said power from outside the patients body.
139. The method according to item 129 or 133, wherein a wireless energy transmitter for wireless transfer of energy is powering said device , to directly during energy transfer, for preventing said transport of an egg, wherein by means of a control device controlling said power from outside the patients body.
140. The method according to item 138, wherein a wireless energy transmitter for wireless transfer of energy is charging said internal energy source, wherein by means of a control device controlling said power from outside the patients body..

### Method restriction embodiments

141. The method according to any of item 97-140, wherein said restriction device comprises a mechanical restriction device.
142. The method according to any of item 97-128, wherein said restriction device comprises hydraulic restriction device.
143. The method according to item 142 wherein said hydraulic restriction device comprises a reservoir, for moving gas or fluid to or from said restriction device.
144. The method according to item 143, wherein said reservoir is placed subcutaneously for
   i. being reached by the patients hand for
   ii. moving fluid manually to or from said restriction device.
145. The method according to any of item 97-128, wherein said restriction device comprises a stimulation device.
146. The method according to any of item 97-128, wherein said restriction device comprises a stimulation device in combination with a mechanical or hydraulic restriction device.

### Method power and remote control

147. The method according to any of item 97-146, wherein said restriction device - being powered by an internal energy source for restricting or releasing said restriction device, said power being controlled from outside the patients body.
148. The method according to any of item 97-146, comprising wireless energy transmitter for transmitting wireless energy wherein said internal energy source being charged by said wireless energy, said wireless power being controlled from outside the patients body.
149. The method according to any of item 97-146, wherein a wireless energy transmitter for wireless transfer of energy is powering said restriction device to, directly during energy transfer, restricting or releasing said restriction device, said power being controlled from outside the patients body.
150. The method according to any of item 97-146, wherein a magnetic field is powering said restriction device for restricting or releasing said restriction device, said power being controlled from outside the patients body.
151. The method according to item 147, wherein the internal source of energy comprising a battery or capacitor for powering said device.
152. The method according to item 147 or 149 , wherein the internal energy source is controlled from outside the patients body.
153. The method according to item 152, wherein the internal energy source is controlled with a wireless remote control.
154. The method according to item 152 or 153, wherein the internal energy source is controlled with a switch implanted in the human body adapted to be regulated from outside the body.

### Control and sensor input

155. The method according to any of item 97-154, comprising a control device to control the restriction device.
156. The method according to item 155, wherein the control device is adapted to control the restriction device from outside the body of the patient.
157. The method according to item 155, wherein the control device are adapted to be operated from outside the human body.
158. The method according to item 155, wherein the control device comprises an internal control unit adapted to be implanted in the body of the patient, wherein the control of the restriction device is made by said internal control unit.
159. The method according to item 158, comprising a sensor sensing at least one physical parameter of the patient.
160. The method according to item 159, wherein the internal control unit controls the restriction device to restrict said oviduct based on information from said sensor.
161. The method according to item 156, comprising a sensor sensing at least one physical parameter of the patient, wherein the control device controls the restriction device based on information from said sensor.
162. The method according to item 158, comprising a sensor sensing at least one functional parameter of the device.
163. The method according to item 162, wherein the internal control unit controls the restriction device to restrict said oviduct based on information from said sensor.
164. The method according to item 156, comprising a sensor sensing at least one functional parameter of the device, wherein the control device controls the restriction device based on information from said sensor.
165. The method according to item 159 or 161. wherein said sensor sensing a hormone level or a temperature.
166. The method according to item 162 or 164, wherein said sensor sensing a electrical parameter or a pressure related to the device.
167. The method according to any of item 97-146, comprising an energy source powering the restriction device, wherein said energy source being an internal energy source adapted to being chargeable by wireless energy, and further comprising a transmitter for transmitting wireless energy to charge said internal energy source.
168. The method according to any of item 97-167, adapted to send feedback information from inside the body to the outside thereof to give feed back related to any functional parameter of the device or physical parameter of the patient.
169. The method according to item 167, adapted to send feedback information from inside the body to the outside thereof to give feed back related to any functional parameter of the device or physical parameter of the patient.
170. The method according to item 169, wherein the functional parameter of the device is correlated to the transfer of energy for charging the internal energy source.
171. The method according to item 170, wherein the functional parameter of the device is the energy balance, the balance between the energy received and the energy used including accumulated by the device.
172. The method according to item 171, wherein the energy balance is the balance between an energy reception rate and an energy using including accumulating rate.
173. The method according to any of item 155-172, wherein the control device comprises a mechanical control device.
174. The method according to any of item 155-172, wherein the control device comprises a hydraulic control device.
175. The method according to any of item 155-172, wherein said control device comprises a stimulation device.

## Claims

1. A pregnancy control system for affecting pregnancy of a female mammal or human patient comprising;
an implantable stimulation device adapted to stimulate a wall of an oviduct of the patient to cause contraction thereof;
wherein the implantable stimulation device is operable to accumulate at least one egg released from the ovary in the oviduct and to cease restricting the oviduct lumen to admit free passage in the oviduct.

2. The system according to claim 1, wherein the implantable stimulation device is adapted to stimulate the oviduct wall to completely restrict the oviduct lumen.

3. The system according to claim 1 or 2, wherein the implantable stimulation device comprises two or more restriction areas adapted to cause constriction of the oviduct wall at different positions on the oviduct.

4. The system according to claim 3, wherein the implantable stimulation device is adapted to restrict the oviduct lumen in the form of a peristaltic wave restriction, moving towards the ovary to prevent passage in the oviduct.

5. The system according to claim 3 or 4, wherein the implantable stimulation device is adapted to restrict the oviduct lumen in the form of a peristaltic wave restriction moving towards the uterus to effect transportation of the egg to the uterus.

6. The system according to any one of the preceding claims, further comprising a control device operable from outside the patient's body to control the implantable stimulation device.

7. The system according to claim 6, wherein the control device further comprises an internal control unit adapted to be implanted in the patient's body, wherein the control of the implantable stimulation device is made by said internal control unit.

8. The system according to any one of the preceding claims, further comprising an implantable internal energy source for powering the implantable stimulation device.

9. The system according to claim 8, wherein the implantable internal energy source is chargeable by wirelessly transmitted energy.

10. The system according to claim 8 or 9, wherein the implantable internal energy source is controllable from outside the patient's body.

11. The system according to any one of the preceding claims, comprising a sensor sensing at least one physical parameter of the patient.

12. The system according to any one of the preceding claims, comprising a sensor sensing at least one functional parameter of the system.

13. The system according to claim 11 or 12, when dependent on claim 7, wherein the internal control unit controls the implantable stimulation device to restrict said oviduct based on information from said sensor.

14. The system according to any one of claims 11 to 13, further comprising a feedback device adapted to send feedback information from inside the patient's body to the outside thereof to give feedback related to the at least one functional parameter of the system or the at least one physical parameter of the patient.

15. The system according to claim 14, wherein the at least one functional parameter of the system is correlated to the transfer of energy for charging the internal energy source.
